# EUROPEAN PATENT SPECIFICATION

(11) **EP 4 153 282 B1**
(45) Date of publication and mention of the grant of the patent: **24.06.2026**
(21) Application number: 21722512.7
(22) Date of filing: 05.05.2021
(51) Int. Cl.: A61M 11/04, A24F 40/00, A61M 15/06

(54) **MOBILE INHALER FOR SUBSTANCE DELIVERY**
MOBILER INHALATOR ZUR SUBSTANZABGABE
INHALATEUR MOBILE POUR DISTRIBUTION DE SUBSTANCE

(30) Priority: 20.05.2020 EP 20175621
(43) Date of publication of application: 29.03.2023
(73) Proprietor: Smokeless.world GmbH, 82031 Grünwald (DE)
(72) Inventor: KILGER, Daniel, 81369 München (DE); MÜLLER, Patrick, 73092 Heiningen (DE); SEDLMEIER, Michael, 80339 München (DE); BIANCHI, Andrea, 82031 Grünwald (DE); DE CHIRICO BACER, Luca, 82031 Grünwald (DE)
(74) Representative: Stellbrink & Partner Patentanwälte mbB
(86) International application number: PCT/EP2021/061910
(87) International publication number: WO 2021/233686

(56) References cited:
- WO-A1-2016/020675
- WO-A1-2019/104227
- WO-A2-2015/073854

## Description

### Field

The invention generally relates to mobile inhalers. More particularly, the invention can relate to mobile inhalers for nicotine-delivery, also known as e-cigarettes. The invention can be particularly useful for mobile inhalers used for nicotine weaning.

According to Wikipedia of October 2018, an electronic cigarette or e-cigarette is a handheld electronic device that simulates the feeling of smoking. It works inter alia by heating a liquid to generate an aerosol, commonly called a "vapor", that the user inhales. Using e-cigarettes is commonly referred to as vaping. The liquid in the e-cigarette usually comprises nicotine, propylene glycol and/or glycerine, and flavorings. Not all e-liquids contain nicotine.

Such mobile inhalers can also be known as e-cigarettes, e-cigs, EC, electronic nicotine delivery systems (ENDS) or electronic non-nicotine delivery systems (ENNDS), electronic smoking devices (ESDs), personal vaporizers, or PVs. They are handheld devices, often made to look like conventional cigarettes, and used in a similar way.

There are three main types of e-cigarettes: cigalikes, looking like cigarettes; eGos, bigger than cigalikes with refillable liquid tanks; and mods, assembled from basic parts or by altering existing products. As the e-cigarette industry continues to evolve, new products are quickly developed and brought to market. A common power source for these inhalers are rechargeable batteries, which are often mounted to the device and recharged in the mounted state. Often lithium-ion batteries are used for this purpose. The U.S. Fire Administration identified such batteries to be the most frequent reason for fires that originated from e-cigarettes and identified 195 fire and explosion incidents relating to e-cigarettes in the US in the time between January 2009 and December 2016. 80 of these incidents resulted in moderate, 38 in severe injuries, cf. McKenna, L. A. "Electronic Cigarette Fires and Explosions in the United States 2009-2016", National Fire Data Center, U.S. Fire Administration (2017) (https://www.usfa.fema.gov/downloads/pdf /publications/electronic_cigarettes.pdf*)* Of these 195 incidents, only 18 occurred when the device was stored. Apart from some incidents where the status of the device was not reported, the majority of incidents occurred when the devices were in use or in the user's pocket - so most probably used before.

The main components of an e-cigarette are typically a mouthpiece, at least one (mountable or integrated) tank, also called a cartridge, a heating element/atomizer, a microprocessor or microcontroller, a battery, and possibly an LED light on the end. The only exception to this are mechanical e-cigarettes (mods) which contain no electronics; a circuit of the heating element is controlled by a mechanical action switch. An atomizer comprises a small heating element, or coil, that vaporizes e-liquid, and wicking material that draws liquid onto the coil. When the e-cigarette is activated, e.g. by the user pushing a button or activating a pressure sensor by inhaling, the heating element atomizes the liquid solution. The e-liquid reaches a temperature of roughly 100-250 °C within a chamber to create an aerosolized vapor, which the user then inhales. The aerosol provides a flavor and feel similar to tobacco smoking. Also, e-cigarettes using a combination of vapor and spray are disclosed, e.g. in WO2015079197A1.

E-cigarettes may be used with other substances and cartridges can potentially be filled with e-liquid containing substances other than nicotine, thus serving as a new way to deliver other psychoactive drugs, for example cannabis.

The emergence of e-cigarettes has given cannabis smokers a new method of inhaling cannabinoids. E-cigarettes, also known as vape pens, cartridges and pens, differ from traditional marijuana cigarettes in several respects. It is assumed that vaporizing cannabinoids at lower temperatures is safer because it produces smaller amounts of toxic substances than the hot combustion of a marijuana cigarette.

The contents of the vapors generated by e-cigarettes are associated with different health risks. For example, microbial toxins are reported to have been found in liquids used for inhalation with e-cigarettes, cf. Christiani, D.C. & Lee, M. "Microbial Toxins in Nicotine Vaping Liquids" in American Journal of Respiratory and Critical Care Medicine, 201(6), pp. 741-743 (2019*).* Also, different metals and other potentially hazardous chemical substances have been identified in vapors from different e-cigarettes, cf. Williams, M., Li, J. & Talbot, P. "Effects of Model, Method of Collection, and Topography on Chemical Elements and Metals in the Aerosol of Tank-Style Electronic Cigarettes" Sci Rep 9, 13969 (2019*).* Even though this study focussed on so-called "tank-style" e-cigarettes, it is expectable that the same principles apply to cartridge-based e-cigarettes, too. Furthermore, problems resulting from "home-made" liquids, that is, liquids mixed by users from legal or illegal components, are reported. The same applies to modifications of the inhalation devices. Particular problems are also reported when it comes to the consumption of illegal drugs by vaporisation, cf. Varlet V. "Drug Vaping: From the Dangers of Misuse to New Therapeutic Devices" Toxics, 4(4), 29 (2016*).*

Particularly after the outbreak of lung injuries related with the use of e-cigarettes in 2019, the composition of the liquids to be vaporised has been discussed again, cf. *"*Outbreak of Lung Injury Associated with the Use of E-Cigarette, or Vaping, Products" Centers for Disease Control and Prevention, https://www.cdc.gov/tobacco/ basic_information/e-cigarettes/severe-lung-disease.html, retrieved on April 22, 2020*.* Also, quality deviations in liquids commercially available have been alleged, stating that contaminated or expired cartridges had been shipped, cf. Kaplan, S. & Hoffman, J. "Juul Knowingly Sold Tainted Nicotine Pods, Former Executive Says", The New York Times, 30.10.2019, updated 20.11.2019, https://www.nytimes.com/2019/10/30/health/juul-pods-contaminated.html, retrieved on April 22, 2020*.*

US 2013/0284192 teaches an electronic cigarette ("e-Cig") including a controller for providing various operations within an e-Cig. Enhancements for the controller may provide for improved operations and control for the e-Cig. In one embodiment, there may be a communications capability that may allow for the e-Cig to communicate with a consumer device. The consumer may then control smoke properties, monitor operations, adjust settings, and/or receive product notifications or offers through the consumer device's communication with the e-Cig. The communications may enable connections to various websites on the Internet for usage tracking or social networking.

US 2012/0048266 teaches an apparatus including a first cartridge, a sensor, and a controller. The first cartridge can include a first release device configured to release a first substance into a housing. The controller can be configured to receive data from the sensor. The controller can determine an amount of first substance released by the first cartridge based on the data. The first release device can be controlled based on the determined amount of first substance.

US 688655782 teaches an inhalation device configured to programmably emit small droplets of multiple components in which quantities of the multiple components can vary with each successive activation of the inhalation device.

WO 2016/005602 teaches an electrically operated aerosol-generating system comprising an aerosol-generating device, and first and second removable aerosol-forming cartridges each comprising a resistive heater. The first removable aerosol-forming cartridge comprises a first aerosol-forming substrate requiring a first heating profile and the second removable aerosol-forming cartridge comprises a second aerosol-forming substrate requiring a second heating profile. The aerosol-generating device comprises a main body defining a cavity and at least one opening for removably receiving one of the first and second aerosol-forming cartridges in the cavity. The aerosol-generating device also comprises an electrical power supply and a control unit for controlling a supply of electrical current from the electrical power supply to the electric heater. The control unit is arranged to detect whether the first or second aerosol-forming cartridge has been received within the cavity based upon the resistive load of the respective resistive heater. The control unit is further arranged to control the supply of electrical current to the at least one electric heater according to either the first or the second heating profile in response to the detected aerosol- forming cartridge.

US 2017/0027229 teaches a method comprising determining an average amount of vapor associated with an average inhalation by a user of an electronic vapor device, determining a quantity of vaporizable material stored in the electronic vapor device, determining a number of inhalations remaining based on the average amount of vapor associated with the average inhalation and the determined quantity of vaporizable material, and displaying the number of inhalations remaining.

WO 2017/205692A1 teaches vaporizers and vaporizer systems, which can include a device in communication with a vaporizer, can include one or more features related to control of functions and/or features of the vaporizer, identification of a cartridge and/or a vaporizable material in the cartridge, data exchange (either one-way or two-way) between a cartridge and a vaporizer with which the cartridge is engaged, and the like.

US 2014/0014126 teaches an electronic cigarette ("e-Cig") which may include functionality for monitoring and controlling the thermal properties of the e-Cig. The system and method described therein may monitor a temperature based on a resistor (i.e. hot wire) near the wick and model the thermal cycle of an e-Cig. The model can be used for controlling the temperature of the e-Cig and preventing burning. The temperature control may dictate optimal conditions for atomization and smoke generation in an e-Cig while avoiding hotspots and burning to the atomizer or cartomizer.

While the prior art approaches may be satisfactory in some regards, they have certain shortcomings and disadvantages. In particular, known and marketed can present a hazard to the user or present an abnormal behavior, for example due to faulty parts or faulty operation of the inhaler. This may be particularly disadvantageous for example in a case where the inhaler is used for nicotine weaning or drug delivery.

### Summary

It is therefore an objective of the present invention to provide a mobile inhaler with an improved fail safety.

It is another optional objective of the present invention to provide a mobile inhaler with reduced hazards for a user of the inhaler.

It is another optional objective of the present invention to limit an unexpected behaviour of a mobile inhaler.

It is another optional objective of the present invention to provide a mobile inhaler with a more predetermined behaviour in case of faulty parts and/or faulty operation.

In a first embodiment, a mobile inhaler is disclosed. The mobile inhaler comprises a power source and at least one aerosolization system.

The power source may comprise a battery. The power source may be rechargeable, such as a rechargeable battery. The power source may be configured for storing electric energy. For example, the power source may comprise a lithium-ion battery. However, the power source may also comprise a capacitor.

The mobile inhaler may be configured for providing a substance for inhalation by a user. The mobile inhaler may be portable, that is, the mobile inhaler may be configured to be carried away by a person. The mobile inhaler may further be configured to be used while being held in at least one hand of a user.

The mobile inhaler may be a handheld device.

The at least one aerosolization system may be configured for aerosolization of at least one liquid. In this disclosure, aerosolization is intended to refer to generating a vapor, mist, spray, aerosol, or a mixture thereof from the at least one liquid.

The mobile inhaler may further comprise an aerosolization system control component. The aerosolization system control component may be configured for controlling the at least one aerosolization system.

The aerosolization system control component may comprise a data-processing system. The aerosolization system control component may comprise at least a portion of a micro-controller. The aerosolization system control component may also comprise at least a portion of a micro-processor. The aerosolization system control component may also comprise a portion of a circuit, such as an integrated circuit (IC) or a printed circuit board (PCB).

The aerosolization system control component may comprise an electronic control element. The electronic control component may be configured for controlling at least a portion of the at least one aerosolization system.

The electronic control element may comprise a transistor, such as a MOS transistor. The transistor may be configured for switching power that is provided to the at least one aerosolization system or to a component thereof.

The aerosolization system control component may also comprise an element like a switch or a relay configured to control whether or much electrical power is provided to the at least one aerosolization system.

The mobile inhaler may comprise at least one reservoir. Each of the at least one reservoir may be configured for holding a liquid to be aerosolized. The reservoir may further comprise a gas, such as air that can be pressurized.

Each aerosolization system may comprise at least one reservoir. The reservoir and the aerosolization system may be integrated. Thus, for example, the reservoir may be integrated into the aerosolization system, so that another element of the aerosolization system is located within the reservoir.

The mobile inhaler may be configured for aerosolizing a liquid comprising at least one of nicotine and an active pharmaceutical ingredient. That is, if the mobile inhaler is configured for aerosolizing more than one liquid, then at least of the liquids may comprise the at least one nicotine and the active pharmaceutical ingredient. The active pharmaceutical ingredient can be a drug, such as a drug for pain medication, e.g. an opioid. However, the active pharmaceutical ingredient can also comprise other pharmacologically active substances, such as tetrahydrocannabinol or other psychoactive substances.

In such embodiments, the other liquid(s) may or may not comprise nicotine and/or an active pharmaceutical ingredient, or some of them may comprise said at least one of said substances, and others may not.

The mobile inhaler may further comprise a control component.

The control component may comprise a data-processing system. The control component may comprise a micro-controller. The control component may also comprise a micro-processor.

The control component may also comprise a portion of a circuit, such as an integrated circuit (IC) or a printed circuit board (PCB). The control component may be a processing unit or a system-on-chip that can be interfaced with the mobile inhaler.

The control component may also comprise further means of data processing, such as, processor units and/or hardware accelerators. The control component may comprise memory components, such as, main memory (e.g. RAM), cache memory (e.g. SRAM) and/or secondary memory (e.g. flash memory, HDD, SDD). The control component can comprise busses configured to facilitate data exchange between components of the control component, and/or to facilitate data exchange between the control component and other components of the mobile inhaler.

The aerosolization system control component may comprise a portion of the control component. In other words, a portion of the control component may perform at least a part of a function of the aerosolization system control component.

The mobile inhaler may further comprise a voltage control component.

The voltage control component may comprise a data-processing system. The voltage control component may comprise at least a portion of a micro-controller. The voltage control component may also comprise at least a portion of a micro-processor. The voltage control component may also comprise a portion of a circuit, such as an integrated circuit (IC) or a printed circuit board (PCB).

The voltage control component may comprise a portion of the control component. In other words, a portion of the control component may perform at least a part of a function of the voltage control component.

The mobile inhaler can comprise a data transmission component. The data transmission component can be configured for sending data and/or receiving data. The data transmission can be configured for wireless data transmission for example by means of electro-magnetic radiation.

Particularly, the data transmission component can be configured to connect the mobile inhaler to a network, such as the internet. For example, the data transmission component can be configured for connecting the mobile inhaler to a smart phone by means of a wireless connection according to the bluetooth standard, or to a router by means of a WiFi-connection.

The data transmission component can also be configured for RFID-communication, for example as specified in ISO/IEC 18000.

The data transmission component may comprise an antenna configured for sending and/or receiving data. The data transmission component may comprise a data-processing system. The data transmission component may comprise at least a portion of a microcontroller. The data transmission component may also comprise at least a portion of a micro-processor. The data transmission component may also comprise a portion of a circuit, such as an integrated circuit (IC) or a printed circuit board (PCB).

The data transmission component may comprise a portion of the control component. In other words, a portion of the control component may perform at least a part of a function of the data transmission component.

The at least one aerosolization system may comprise at least one vaporization system. The at least one vaporization system may be configured for heating of the at least one liquid to be aerosolized. The vaporization system may be configured for vaporizing the at least one liquid.

Each aerosolization system may be a vaporization system. In other words, the at least one aerosolization system may be at least one vaporization system.

The at least one vaporization system may be configured for heating of the liquid to be aerosolized. The at least one vaporization system may be configured for vaporizing the liquid at least by heating it. The vaporization may also be based on a combination of effects, as disclosed e.g. in US 2013/0284192.

Each vaporization system may be configured for thermal aerosolization. That is, each of the at least one vaporization system may be configured for aerosolization of the respective liquid by heating the respective liquid. However, this is intended to not exclude that other mechanisms may contribute to aerosolization, too, such as the effects associated with a use of a wick or other capillary elements.

Each vaporization system may comprise a heating unit, such as a heating coil or a ceramic heating element. The heating unit may be configured for converting electric energy to heat. The heating unit may be controllable.

As the person skilled in the art will easily understand, the heating unit may be in use together with a wick for facilitation of the vaporization.

Each aerosolization system may comprise a connection to the power source.

The mobile inhaler may comprise a fault detection component.

The fault detection component may comprise a data-processing system. The fault detection component may comprise at least a portion of a micro-controller. The fault detection component may also comprise at least a portion of a micro-processor. The fault detection component may also comprise a portion of a circuit, such as an integrated circuit (IC) or a printed circuit board (PCB).

The fault detection component may comprise a portion of the control component. In other words, a portion of the control component may perform at least a part of a function of the fault detection component.

The fault detection component may be configured for detecting a fault, particularly a fault of the mobile inhaler.

The fault detection component may be configured for deactivating the at least one aerosolization system. This may be optionally advantageous as it may allow for a secure and predetermined behavior of the mobile inhaler in case of a fault. Furthermore, it may be optionally advantageously increasing a chance of the mobile inhaler failing to safety, as for example an overheating of the aerosolization or a power consumption of the aerosolization system in case of a fault may be inhibited.

In this disclosure, deactivating the at least one aerosolization system or one of the aerosolization system is intended to refer to bringing the one or the a least one system in a state where it cannot be actuated. For example, an electrical connection from the power source to the respective aerosolization system can be interrupted, e.g. by means of a switch, a fuse, a transistor, a relay or a resistor providing a sufficiently high resistance.

In this disclosure, activating the at least one aerosolization system or one of the aerosolization system is intended to refer to bringing the aerosolization system or the at least one aerosolization system in a state where it can be actuated. In other words, an activated aerosolization system is intended to refer to an aerosolization system that can be actuated, e.g. by a user or by the mobile inhaler, and a deactivated aerosolization system is intended to refer to an aerosolization system that cannot be actuated in its current state.

The fault detection component can be configured for deactivating at least a part of the aerosolization control system. For example, the fault detection component can be configured for setting a switch, relay or transistor of the aerosolization control system to a state where no power is provided to the aerosolization system.

The fault detection component may also be configured for deactivating the power source. For example, the fault detection component may be configured for interrupting an electrical connection of the power source to at least one element of the mobile inhaler, preferably a remainder of the mobile inhaler. This may be optionally advantageous, as damages to the power source may be avoided in case of a fault. In cases where the power source comprises the battery, particularly the lithium ion battery, it can be avoided that the power source heats up overly. Also, it can be optionally advantageously avoided that the power source takes fire, for example in cases where a short-circuit fault occurs.

The fault detection component may be configured for operating based on a temperature. In other words, the fault detection component may be configured for detecting the fault based on said temperature. This may be optionally advantageous, since a temperature can be a reliable and simple way of detecting problems in electronic systems.

The fault detection component may be configured for operating based on a temperature of the power source. This may be optionally advantageous as certain types of power sources, for example lithium ion batteries, tend to heat up in case that an internal fault occurs. Also, short-circuit faults tend to lead to an increased temperature of a power source connected to the short-circuit. Hence, short-circuit faults in the whole inhaler may be detected.

The fault detection component may be configured for operating based on a temperature of a housing of the mobile inhaler. This can be optionally advantageous as it may allow to avoid dangerous or inadmissible heating of components of the inhaler which are in contact with the user or an environment.

The fault detection component may be configured for performing the deactivating upon detection of the fault.

The fault detection component may comprise at least one of a positive temperature coefficient element and a negative temperature coefficient element. The positive temperature coefficient element may for example be arranged in series with all electric consumers connected to the power source, or at least in series with the at least one vaporization system. The negative temperature coefficient element may for example be arranged so as to inhibit a provision of power to the at least one vaporization system once a fault is detected.

The fault detection element may further be optionally advantageous, as it may protect at least one of the aerosolization system, the vaporization system and the heating unit, from overheating. Furthermore, a result "dry hit", that is, gas provided to a user when for example the wick runs dry, may be avoided in case of a fault leading to a permanent actuation of the heating element.

The mobile inhaler may be configured for operating according to an operation mode depending on a voltage provided by the power source. In other words, the mobile inhaler may operate components or elements differently and/or may operate different elements or components depending on a voltage provided by the power source.

The voltage control component may be configured for sensing a voltage provided by the power source. The voltage control component may comprise a voltage sensing element.

The voltage control component may be configured for deactivating the aerosolization system, if the voltage provided by the power source is below a lower threshold.

The voltage control component may be configured for reducing a voltage provided to the at least one aerosolization system, if the voltage provided by the power source is above an upper threshold. The upper threshold may be an absolute value.

The upper threshold may be relative to a maximum voltage provided by the power source in a full state, for example if the power source comprises a battery. In such cases, the upper threshold may for example be in a range between 75% and 95% of the maximum voltage provided by the power source.

The lower threshold may be relative to the maximum voltage provided by the power source in a full state. In such cases, the lower threshold may for example be in a range between 40% and 70% of the maximum voltage provided by the power source.

The maximum voltage provided by the power source may be a voltage that the power source provides at a state of charge of 100%, if the power source comprises a battery.

The mobile inhaler may be configured for substantially providing the voltage provided by the power source to the aerosolization system, if the voltage provided by the power source is between the lower and the upper threshold.

The voltage control component may be configured for reducing the voltage provided to the aerosolization system by means of the aerosolization system control component. For example in cases where the aerosolization system control component comprises a transistor, the voltage control component may be configured for reducing the voltage provided to the aerosolization system by a pulse width modulation of on and off cycles of the transistor.

The voltage control component may be optionally advantageous, as it may lead to a limitations of deviations of a voltage provided to the at least one aerosolization. Thus, the aerosolization may be steadier over a state of charge of the power source, particularly if the aerosolization depends on the voltage provided to the aerosolization system.

For example in case of a heating element using the principle of resistive heating, such as the above discussed heating unit, a thermal power provided may depend on a current flowing through the heating unit and thus also on a voltage provided to the heating unit. Thus, the above-described technology may also optionally advantageously reduce a deviation of a temperature of the heating unit (or heating units, in case of a plurality of aerosolization systems).

As becomes obvious from the above, the voltage control component can be optionally further advantageous, as it may reduce deviations of an amount of aerosolized liquid per time of actuation which may be caused by the temperature of the heating unit of the at least one aerosolization system.

Furthermore, optionally advantageously, a temperature limitation of the heating unit may limit toxins in a provided aerosols, since a rate of emitted toxins may rise if the heating element is operated at a comparably higher power level and higher temperature. (cf. *Williams & Talbot* as well as *Varlet* as indicated above).

Furthermore, the voltage control component may optionally advantageously lead to a predetermined behavior of the mobile inhaler when the voltage provided by the power source deviates, e.g. due to a dependence on the state of charge.

Also, when the voltage provided by the power source drops, the mobile inhaler may optionally advantageously fail to a predetermined state.

The voltage control component may be configured for reducing the voltage provided to the at least one aerosolization system and/or the at least one aerosolization system control component if the fault detection component detects a fault.

The fault detection component may be configured for performing the deactivating by means of the voltage control component.

Thus, optionally advantageously, the fault detection component may not need an element for reducing or interrupting the voltage provided to the at least one aerosolization system/aerosolization control system. In a case where the fault detection component and the voltage control component comprise elements configured for reducing the voltage in case of a fault, a probability of a non-reduction of the voltage despite a detected fault may be optionally advantageously reduced.

The voltage control component may comprise at least one transistor, and preferably a power converter, such as a step-down converter. The transistor may be configured for transmitting and/or switching the power from the power source to at least one of the at least one aerosolization system and the at least one aerosolization control system.

The mobile inhaler may comprise a replacement part.

The replacement part may be a replacement part for regular replacement, that can be, for regular replacement after a certain duration of use or a certain amount of liquid has been vaporized.

The replacement part may be a replacement part for replacement by an end user. For example, the replacement part may be connected to a remainder of the mobile inhaler in releasable way. The replacement part may be configured to be exchanged without a need of tools.

The replacement part may for example be a cartridge.

The replacement part may comprise the at least one aerosolization system.

The replacement part may also comprise at least one of the at least one reservoir.

The replacement part may comprise a data storage component.

The data storage component may be configured for storing data relating to the replacement part. The data storage component may be configured to identify the replacement part.

The data storage component may comprise at least one memory component, such as, main memory (e.g. RAM), cache memory (e.g. SRAM) and/or secondary memory (e.g. HDD, SDD, flash memory). At least one memory component of the data storage component may be read-only-memory.

The data storage component may comprise a data-processing system. For example, the data storage component may comprise at least a portion of a micro-controller. The data storage component may also comprise at least a portion of a micro-processor. The data storage component may also comprise a portion of a circuit, such as an integrated circuit (IC) or a printed circuit board (PCB).

The data storage component may comprise at least one interface for data exchange, such as data exchange with a remainder of the mobile inhaler. The interface can for example be an interface for wired connection, such as a plug. The interface may also be an interface for wireless communication, such as an antenna.

The data storage component may be configured for storing identification data. The identification data may indicate an identity of the replacement part, such as a serial number of the replacement part, a type code of the replacement part, and/or identification data relating to components of the replacement part, e.g. the fluid in the at least one reservoir, the at least one aerosolization system or the data storage component itself. The identification data may also comprise an indicator of a time of production, a batch number or the like.

An optional advantage of storing the identification data may be that they allow for identification of the replacement part.

The identification data may be cryptographically signed.

The data storage component may be configured for storing state data. The state data may relate to a state of the replacement part or an element thereof. For example, if the replacement part comprises the at least one reservoir, the state data may indicate at least one of an amount of remaining liquid, an extrapolation thereof and a state such as "depleted". In a case where the replacement part comprises the at least one aerosolization system, the state data may indicate a time for which the aerosolization system has been used, e.g. a time for which it has been actuated. The state data may also indicate whether the replacement part is faulty or whether an error or fault occurred during its operation.

The data transmission component may be configured for receiving at least one of identification data and state data from the replacement part. Furthermore, the data transmission component may be configured for receiving at least one of identification data and state data from the data storage component.

The data transmission component may be configured for sending instruction data to the replacement part, preferably to the data storage component, upon which the replacement part, and preferably the data storage component, sends the data to the data transmission component.

The mobile inhaler may be configured for activating the replacement part only if the identification data match an identity-criterion. The identity-criterion may for example relate to a valid cryptographic signature of the identification data. The identity-criterion may also relate to whether an identity indicated by the identification data belongs to a list or group of identities, such as a list of identities associated with replacement parts manufactured under controlled conditions. Thus, for example in case of a quality problem in production or problems with parts or liquids manufactured in a same batch, it may be possible to inhibit a usage of said parts to avoid harm to the user, e.g. in case of a contaminated liquid, or damage to the remainder of the mobile inhaler, e.g. in case of a short-circuit in the aerosolization system.

The mobile inhaler may be configured for activating the replacement part only if the state data match a state-criterion. The state-criterion may for example relate to an absence of data indicating a fault of the replacement part. The state-criterion may also relate to the time for which the aerosolization system has been actuated or to the amount of remaining liquid. An optional advantage of the state criterion may be that the replacement part may not be activated in cases that could result in damage of the mobile inhaler, malfunction, in cases where the reservoir may be empty, or in cases where the aerosolization system may have reached an end of service life. Thus, optionally advantageously, generating vapour comprising hazardous or toxic matter because of an empty reservoir or an overly aged aerosolization system may be inhibited.

The mobile inhaler is configured for outputting an indicator of at least one of the matching of the state-criterion and the identity-criterion. Outputting the indicator can comprise transmitting the indicator to another device, such as to a smart phone, where the indicator is outputted subsequently.

The mobile inhaler may be configured for receiving at least one of the identity-criterion and the state-criterion from a server. An optional advantage may be that the criteria may be updated, e.g. if problems with certain replacement elements were detected, data storage components went lost or were tampered with, or quality deviations where detected, even if the replacement parts were already distributed.

The data transmission component may be configured for sending data to the replacement part. The data transmission may be configured for sending the data to the replacement part by means of a wireless connection, such as by RFID-communication, as discussed above. The data transmission component may also be configured for sending the data to the replacement part by means of a wired connection.

The data transmission component may be configured for sending data to the data storage component.

The data transmission component may be configured for sending data relating to a usage of the mobile inhaler to the replacement part, preferably to the data storage component.

The data transmission component may be configured for sending data relating to an amount of dispensed substance from the at least one reservoir of the replacement part. The data relating to the at least one reservoir of the replacement part may for example be a volume of vaporized liquid, a number of seconds that the aerosolization system comprising the reservoir was activated and dispensed substance from this reservoir, or a number of puffs that the user took from the corresponding reservoir. The mobile inhaler may be configured for determining or estimating said data relating to the amount of dispensed substance, as discussed e.g. in US 2012/0048266 A1.

The data transmission component may be configured for sending data relating to a use of the at least one aerosolization system of the replacement part. The data relating to the use of the aerosolization system may for example relate to at least one of a time of actuating of the aerosolization system, an amount of liquid vaporized by the aerosolization system and an amount of energy consumed by the aerosolization system. The mobile inhaler may be configured for determining or estimating said data relating to the use of the at least one aerosolization system.

The data transmission component may be configured for sending state data to the replacement part. Particularly, the data transmission component may be configured for sending state data to the data storage component.

The state data may comprise at least a portion of the data relating to the amount of dispensed substance from the at least one reservoir. The state data may also comprise at least a portion o the data relating to the use of the at least one aerosolization system.

The data transmission component may be configured for sending state data to the replacement part which do not match the state-criterion based on the data relating to the amount of dispensed substance. In other words, the data transmission component may be configured for sending data to the replacement part which inhibit activation of the replacement part, based on the data relating to the amount of dispensed substance. For example, the data transmission component may send said data if the mobile inhaler estimates at least one of the at least one reservoir of the replacement part to be depleted.

The data transmission component may be configured for sending state data to the replacement part which do not match the state-criterion based on the data relating to the use of the at least one aerosolization system. For example, the data transmission component may send such state data in a case where the at least one vaporization system was actuated for a time corresponding to a maximum service life.

The data transmission component may be configured for sending state data to the replacement part which do not match the state-criterion if the fault detection component deactivates the aerosolization system. In other words, the mobile inhaler may be configured for sending such state data if a fault was detected in the mobile inhaler. This may be optionally advantageous to avoid reuse of unverified replacement parts after a fault occurred. Hence, optionally, a reuse of potentially damaged replacement parts may be inhibited.

The data storage component and the data transmission component may be configured for data exchange by a wireless communication, such as a connection by electro-magnetic radiation. As discussed above, the wireless communication may be according to ISO/IEC 1800. However, the wireless communication may also be using another technology for wireless data transmission.

The data storage component may for example comprise an antenna configured for detecting and/or generating electro-magnetic radiation for the wireless communication.

The data transmission component may also comprise an antenna configured for detecting and/or generating electro-magnetic radiation for the wireless communication.

The data storage component may comprise an identification unit. The identification unit may for example comprise an RFID-tag. The identification unit may be configured for identification of the replacement part. The identification unit may comprise a unique data element, such as a serial number. The identification unit may be configured for encrypted communication.

The identification unit may be configured for storing at least one of the state data and the identification data.

The data storage component may comprise an adhesive layer fixing the identification unit to the housing of the replacement part. In other words, the data storage component may comprise an adhesive layer, wherein the identification unit is attached to the housing of the replacement part by means of the adhesive layer.

The data storage component may comprise a surface layer. The surface layer may be configured for covering the identification unit. In other words, the surface layer may be placed on an outer side of the identification side, that is, on a side facing away from the replacement part.

The surface layer may be made from a non-metallic material. The surface layer may be substantially made from a polymer or a polymer composition. This may be optionally advantageous, since in such a case, the surface layer may only have a negligible or no impact on electro-magnetic radiation received or sent by the identification unit.

The configured to protect the identification unit from mechanical influences, such as scratches.

The data storage component may comprise a further adhesive layer fixing the surface layer at least partially on the identification unit. In other words, the data storage component may comprise a further adhesive layer, wherein the surface layer is at least partially attached to the identification unit of the replacement part by means of the further adhesive layer.

However, in some cases, the data storage component may only comprise the further adhesive layer and the adhesive layer.

The data storage component and the data transmission component may also be configured for data exchange by an electrical connection.

In other words, the data storage component may be configured for data exchange a wired connection. Also, the data transmission component may be configured for data exchange by the wired connection. The data storage component and the data transmission component may be configured for exchanging data by means of the wired connection.

The replacement part may comprise contacts. The contacts may be configured for electrically connecting the replacement part to the remainder of the mobile inhaler.

Particularly, in cases where the replacement part comprises the at least one aerosolization system, the contacts may be configured for electrically connecting the at least one aerosolization system to the remainder of the mobile inhaler.

The data storage component and the data transmission component may be configured for data exchange by means of the contacts (26). Such data exchange by means of electrical contacts is for example discussed in detail in WO 2017/153589 A1 and as well as in WO 2015/138560A1*.*

The identification unit may be permanently attached to the replacement part. In other words, the identification unit may be connected in a non-releasable way to the replacement part. For example, the identification unit may be glued to the replacement part. The identification unit may also be an integral part of the replacement part, for example if the replacement part is injection-moulded and the identification unit is already inserted already in the moulding process.

The identification unit may be configured to be destroyed when removed from the replacement part. For example, a structural integrity of the identification unit may be lower than an adhesion of the identification unit to the replacement part which is generated by an adhesive attaching the identification unit to the replacement part.

An optional advantage of permanently attaching the identification unit and/or the identification unit be configured to be destroyed when removed when removed may be that tampering with the data storage components and/or identification units may be inhibited. Optionally, a reuse of identification elements may be inhibited.

The at least one aerosolization system may be at least two aerosolization systems. Each of the at least two aerosolization systems may be configured for aerolizing a different liquid. The liquids may differ in their components. They may also differ in their quantitative composition, i.e. in the relative amounts of their components.

One of the aerosolization systems may be a first aerosolization system, and one may be a second aerosolization system.

For example, if a first liquid comprises a first concentration of nicotine and/or the active pharmaceutical substance, then, a second liquid may comprise a second concentration of nicotine and/or the active pharmaceutical ingredient. However, the second liquid may also not comprise nicotine and/or the active pharmaceutical substance at all, or it may comprise a different substance.

The at least one reservoir may be at least two reservoirs. Each reservoir can be configured for holding a different liquid to be aerosolized. Each aerosolization system may comprise at least one of the at least two reservoirs. For example, each aerosolization system may comprise one of the at least two reservoirs.

The mobile inhaler may comprise a second aerosolization system control component. The second aerosolization system control component may be configured for controlling the second aerosolization system.

In some embodiments, one of the liquids may comprise at least one substance selected from nicotine and the active pharmaceutical ingredient, and one of the liquids may not comprise said substance.

One of the at least two aerosolization systems may comprise a heating unit. This aerosolization system may be configured for thermal aerosolization. The other aerosolization system may be configured for nebulization.

The fault detection component may be configured for deactivating the at least two aerosolization systems. This may be optionally advantageous particularly in a case where a fault cannot clearly be attributed to one of the two aerosolization systems or does not occur in the aerosolization systems, but in another portion of the mobile inhaler. This may also be optionally advantageous, as a generated vapour is not generated only from the first or only from a second liquid. Thus, optionally, a deviation of a ratio of components of the different liquids in the generated vapour may be avoided.

The fault detection component may be configured for deactivating at least a part of the aerosolization control system and the second aerosolization control system. The above-discussed optional advantages may optionally apply to such an embodiment, too.

The replacement part may comprise the at least two aerosolization systems.

The replacement part may comprise the two reservoirs.

The data storage component may be configured for storing identification data for at least one of each of the aerosolization systems and each of the reservoirs. In other words, the data storage component may be configured for storing identification data for each of the aerosolization systems. The data storage component may also be configured for storing identification data for each of the reservoirs.

The data storage component may be configured for storing state data for at least one of each of the aerosolization systems and each of the reservoirs. That is, the data storage component may be configured for storing state data for each of the aerosolization systems. The data storage component may also be configured for storing state data for each of the reservoirs.

The mobile inhaler may be configured for activating each aerosolization system with the corresponding reservoir only if the corresponding identification data match the identification-criterion.

The person skilled in the art will easily understand that for an example of an aerosolization system A and a reservoir A and an aerosolization system B and a reservoir B, identity data corresponding to aerosolization system A and reservoir A may be identity data relating to at least one of the aerosolization system A and reservoir A. Identity data corresponding to aerosolization system B and reservoir B may be identity data relating to at least one of the aerosolization system B and reservoir B.

This may be optionally advantageous as it inhibits activation of aerosolization systems/reservoirs which do not match the identification-criterion, but it does not necessarily inhibit an activation of other aerosolization systems/reservoirs. Thus, optionally and depending on the liquids in the reservoirs, e.g. one of the liquids may still be vaporized and provided to the user.

Furthermore, an optional advantage can be that, in case of replacement parts that are designed to be refilled/refurbished and that may comprise a significant volume of liquid, only a depleted/faulty reservoir or aerosolization system needs to be refurbished/refilled.

The mobile inhaler may be configured for activating each aerosolization system with the corresponding reservoir only if the corresponding state data match the state-criterion.

The person skilled in the art will easily understand that for an example of an aerosolization system A and a reservoir A and an aerosolization system B and a reservoir B, state data corresponding to aerosolization system A and reservoir A may be state data relating to at least one of the aerosolization system A and reservoir A. State data corresponding to aerosolization system B and reservoir B may be state data relating to at least one of the aerosolization system B and reservoir B.

The data transmission component may be configured for sending data relating to an amount of dispensed substance from each of the two reservoirs of the replacement part. The data relating to the amount of dispensed substance may be according to the above discussion of such data for the at least one reservoir.

The data transmission component may be configured for sending data relating to the use of each of the aerosolization systems of the replacement part. The data relating to the use of each of the aerosolization systems may be according to the above discussion of such data for the at least one aerosolization system.

The data transmission component may be configured for sending the state data for each of the at least one aerosolization systems and each of the reservoirs. That is, the data transmission component may be configured for sending the state data for each of the at least two aerosolization systems. The data transmission component may also be configured for sending the state data for each of the reservoirs.

The data transmission component may be configured for sending these data to the data storage component.

The mobile inhaler may be configured for activating one aerosolization system with the corresponding reservoir only if the corresponding identification data match the identification-criterion, and the other aerosolization system with the corresponding reservoir only if the identification data corresponding to both, that is the at least two, aerosolization systems with the corresponding reservoirs match the identification-criterion.

The identification data corresponding to the at least two aerosolization systems with the corresponding reservoirs matching the identification criterion can refer to the data relating to each of the systems individually matching the identification criterion. It can also refer to the data relating to the at least two systems with the corresponding reservoirs matching the identification-criterion together.

The mobile inhaler may be configured for activating one aerosolization system with the corresponding reservoir only if the corresponding state data match the state-criterion, and the other aerosolization system with the corresponding reservoir only if the state data corresponding to both aerosolization systems with the corresponding reservoirs match the state-criterion.

The state data corresponding to the at least two aerosolization systems with the corresponding reservoirs matching the state-criterion can refer to the data relating to each of the systems individually matching the state-criterion. It can also refer to the data relating to the at least two systems with the corresponding reservoirs matching the state-criterion together.

The above-discussed two embodiments can be optionally advantageous in a case where a vapour of one of the liquids can be provided alone without harm to the user, but the other cannot, or at least, harm resulting from delivering the other liquid alone cannot be excluded. For example, in case of a use of the mobile inhaler for nicotine-weaning purposes, one liquid may comprise nicotine, and the other may not. Depending on a state of a dependency of the user, it may be reasonable to aerosolize only the one, if the aerosolization system for the other does not match the identification criterion. E.g., in a beginning of a weaning phase, aerosolizing only the nicotine-containing liquid may not be overly harmful, while a replacement for a replacement part is underway, however, dispensing only the nicotine-free vapour may lead to strong withdrawal syndromes. In a final part of a weaning phase, aerosolizing only the nicotine-free liquid may be acceptable for a certain time, whereas dispensing only the nicotine-containing liquid could lead to a relapse.

The mobile inhaler may be configured for activating each aerosolization system with the corresponding reservoir only if the identification data corresponding to the at least two aerosolization systems match the identification-criterion.

The mobile inhaler may also be configured for activating each aerosolization system with the corresponding reservoir only if the state data corresponding to the at least two aerosolization systems match the state-criterion.

The above two embodiments may be optionally advantageous if the replacement part is highly integrated, e.g. if the aerosolization systems share at least one component, since a fault or problem of one aerosolization system may impact the other.

The above two embodiments may further be optionally advantageous if both liquids need to be vaporized or if a ratio of the liquids in a resulting aerosol/vapour is relevant in an application of the mobile inhaler. An example may be an application of the mobile inhaler for nicotine weaning. An amount of nicotine in the generated aerosol/vapour may be successively reduced, one of the liquids my comprise nicotine, the other may not comprise nicotine. In this example, only one of the aerosolization systems cannot provide an aerosol/vapour that the at least two aerosolization systems could provide in combination. Thus, it may be detrimental to a weaning process to provide vapour/aerosol from only one of the aerosolization systems.

Also disclosed is a base of the mobile inhaler. Advantages and details discussed in the context of the mobile inhaler may respectively apply also in the context of the base of the mobile inhaler.

The base of the mobile inhaler comprises the power source.

The base of the mobile inhaler may comprise the at least one aerosolization system.

The base of the mobile inhaler may be configured to be connected to the at least one aerosolization system.

The base of the mobile inhaler may further comprise the aerosolization system control component.

The aerosolization system control component may comprises the electronic control element.

The electronic control element may comprise the transistor, such as the MOS transistor. The transistor may be configured for transmitting and/or switching power provided to the aerosolization system.

The base of the mobile inhaler may comprise the at least one reservoir. Each reservoir may be configured for holding a liquid to be aerosolized.

The base of the mobile inhaler may be configured to be connected to the at least one reservoir, wherein the reservoir may be configured for holding liquid to be aerosolized.

Each aerosolization system may comprise at least one reservoir.

The base of the mobile inhaler may be configured for aerosolizing the liquid comprising at least one of nicotine and the active pharmaceutical ingredient, as discussed above.

The base of the mobile inhaler may further comprise the control component.

The base of the mobile inhaler may comprise the voltage control component.

The base of the mobile inhaler may further comprise the data transmission component.

The at least one aerosolization system may comprise the at least one vaporization system. The at least one vaporization system may be configured for heating of the liquid to be aerosolized.

Each aerosolization system may be a vaporization system. That is, the at least one aerosolization system may be the at least one vaporization system.

The at least one vaporization system may be configured for heating of the liquid to be aerosolized.

Each vaporization system may be configured for thermal aerosolization.

Each vaporization system may comprise a heating unit, such as a heating coil or a ceramic heating element.

Each aerosolization system may comprise a connection to the power source.

The base of the mobile inhaler may comprise the fault detection component.

The fault detection component may configured for detecting a fault.

The fault detection component may be configured for deactivating the aerosolization system.

The fault detection component may be configured for deactivating at least a part of the aerosolization control system.

The fault detection component may be configured for deactivating the power source.

The fault detection component may be configured for operating based on the temperature.

The fault detection component may be configured for operating based on the temperature of the power source.

The fault detection component may be configured for operating based on the temperature of a housing of the base of the mobile inhaler.

The fault detection component may be configured for performing the deactivating upon detection of the fault.

The fault detection component may comprise at least one of the positive temperature coefficient element and the negative temperature coefficient element, as discussed above.

The base of the mobile inhaler may be configured for operating according to the operation mode depending on the voltage provided by the power source.

The voltage control component may be configured for sensing a voltage provided by the power source.

The voltage control component may be configured for deactivating the aerosolization system, if the voltage provided by the power source is below a lower threshold.

The voltage control component may be configured for reducing a voltage provided to the at least one aerosolization system, if the voltage provided by the power source is above an upper threshold.

The base of the mobile inhaler may be configured for substantially providing the voltage provided by the power source to the aerosolization system, if the voltage provided by the power source is between the lower and the upper threshold.

The voltage control component may be configured for reducing the voltage provided to the aerosolization system by means of the aerosolization system control component.

The voltage control component may be configured for reducing the voltage provided to the aerosolization system and/or the aerosolization system control component if the fault detection component detects a fault.

The fault detection component may be configured for performing the deactivating by means of the voltage control component.

The voltage control component may comprise at least one transistor, and preferably a power converter, such as a step-down converter. As discussed above, the transistor may be configured to transmit and/or switch power coming from the power source to the at least one aerosolization system and/or the aerosolization control system.

The base of the mobile inhaler may be configured to be connected to the replacement part.

The replacement part may comprise the at least one aerosolization system.

The replacement part may comprise the at least one of the at least one reservoir.

The replacement part may comprise the data storage component.

The data storage component may be configured for storing the identification data.

The data storage component may be configured for storing the state data.

The data transmission component may be configured for receiving at least one of identification data and state data from the replacement part.

The data transmission component may be configured for receiving at least one of identification data and state data from the data storage component.

The base of the mobile inhaler may be configured for activating the replacement part only if the identification data match the identity-criterion.

The base of the mobile inhaler may be configured for activating the replacement part only if the state data match the state-criterion.

The base of the mobile inhaler may be configured for outputting the indicator of at least one of the matching of the state-criterion and the identity-criterion, as discussed above.

The base of the mobile inhaler may be configured for receiving at least one of the identity-criterion and the state-criterion from the server.

The data transmission component may be configured for sending data to the replacement part.

The data transmission component may be configured for sending data to the data storage component.

The data transmission component may be configured for sending data relating to the usage of the mobile inhaler to the replacement part, preferably to the data storage component. The usage of the mobile inhaler may be a usage of the base of the mobile inhaler, or a usage of the mobile inhaler with the replacement part.

The data transmission component may be configured for sending the data relating to the amount of dispensed substance from the at least one reservoir of the replacement part.

The data transmission component may be configured for sending data relating to the use of the aerosolization system of the replacement part.

The data transmission component may be configured for sending state data to the replacement part.

The data transmission component may be configured for sending state data to the replacement part which do not match the state-criterion based on the data relating to the amount of dispensed substance.

The data transmission component may be configured for sending state data to the replacement part which do not match the state-criterion based on the data relating to the use of the aerosolization system.

The data transmission component may be configured for sending state data to the replacement part which do not match the state-criterion if the fault detection component deactivates the aerosolization system.

The data transmission component may be configured for data exchange with the data storage component by the wireless communication, such as the connection by electro-magnetic radiation.

The data storage component may comprise the identification unit, such as the RFID-tag.

The data transmission component may comprise the antenna.

The data transmission component may be configured for data exchange with the data storage component by the electrical connection.

The base of the mobile inhaler may comprise the contacts, and wherein the data transmission component may be configured for data exchange with the data storage component by means of the contacts.

The identification unit may be permanently attached to the replacement part.

The at least one aerosolization system may be least two aerosolization systems, each configured for aerolizing a different liquid. One of the aerosolization systems may be the first aerosolization system, another may be the second aerosolization system.

The at least one reservoir may be at least two reservoirs. Each reservoir may be configured for holding a different liquid to be aerosolized. Each aerosolization system may comprise at least one of the at least two reservoirs. For example, each aerosolization system may comprise one of the at least two reservoirs.

The base of the mobile inhaler may comprise the second aerosolization system control component. The second aerosolization system control component may be configured for controlling the second aerosolization system.

One of the liquids may comprise at least one substance selected from nicotine and the active pharmaceutical ingredient. In such an embodiment, one of the liquids may not comprise said substance.

One of the aerosolization systems may comprise a heating unit and may be configured for thermal aerosolization. The other may be configured for nebulization.

The fault detection component may be configured for deactivating the at least two aerosolization systems.

The fault detection component may be configured for deactivating at least a part of the aerosolization control system and of the second aerosolization control system.

The replacement part may comprise the at least two aerosolization systems.

The replacement part may comprise the at least two reservoirs.

The data storage component may be configured for storing identification data for at least one of each of the aerosolization systems and each of the reservoirs. In other words, the data storage component may be configured for storing identification data for each of the aerosolization systems. The data storage component may also be configured for storing identification data for each of the reservoirs.

The data storage component may be configured for storing state data for at least one of each of the aerosolization systems and each of the reservoirs. In other words, the data storage component may be configured for storing state data for each of the aerosolization systems. The data storage component may also be configured for storing state data for each of the reservoirs.

The base of the mobile inhaler may be configured for activating each aerosolization system with the corresponding reservoir only if the corresponding identification data from the data storage component of the replacement part match the identification-criterion.

The base of the mobile inhaler may be configured for activating each aerosolization system with the corresponding reservoir only if the corresponding state data match the state-criterion.

The data transmission component may be configured for sending the data relating to the amount of dispensed substance from each of the two reservoirs of the replacement part.

The data transmission component may be configured for sending data relating to the use of each of the aerosolization systems of the replacement part.

The data transmission component may be configured for sending the state data for each of the at least one aerosolization systems and each of the reservoirs.

The base may be configured for activating one aerosolization system with the corresponding reservoir only if the corresponding identification data match the identification-criterion, and the other aerosolization system with the corresponding reservoir only if the identification data corresponding to both aerosolization systems with the corresponding reservoirs match the identification-criterion.

The base may be configured for activating one aerosolization system with the corresponding reservoir only if the corresponding state data match the state-criterion, and the other aerosolization system with the corresponding reservoir only if the state data corresponding to both aerosolization systems with the corresponding reservoirs match the state-criterion.

The base may be configured for activating each aerosolization system with the corresponding reservoir only if the identification data corresponding to the at least two aerosolization systems match the identification-criterion.

The base may be configured for activating each aerosolization system with the corresponding reservoir only if the state data corresponding to the at least two aerosolization systems match the state-criterion.

In a third embodiment, a replacement part is disclosed. Above-mentioned advantages, definitions and features as well as further details may apply respectively.

The replacement part comprises the at least one aerosolization system.

The replacement part may be configured for use with a base of a mobile inhaler as discussed above.

Each aerosolization system may comprise at least one reservoir. The at least one reservoir may be according to the above part of the disclosure.

Each reservoir may be configured for holding a liquid to be aerosolized.

At least one of the at least one aerosolization system may be configured for aerosolizing the liquid comprising at least one of nicotine and the active pharmaceutical ingredient.

The replacement part may further comprise the voltage control component.

The replacement part may be a replacement part for the above-disclosed mobile inhaler.

The replacement part may be a replacement part for regular replacement.

The replacement part may be a replacement part for replacement by an end user. For example, the replacement part may be configured to be mounted and unmounted to and from the base of the mobile inhaler without tools.

The at least one aerosolization system may comprise at least one vaporization system. The at least one vaporization system may be configured for heating of the liquid to be aerosolized.

Each aerosolization system may be a vaporization system. Each vaporization system may be configured for heating of the liquid to be aerosolized.

The at least one vaporization system may be configured for heating of the liquid to be aerosolized.

Each vaporization system may be configured for thermal aerosolization.

Each vaporization system may comprise a heating unit, such as a heating coil or a ceramic heating element.

Each aerosolization system may be configured to be connected to the power source.

The replacement part may comprise the fault detection component.

The fault detection component may be configured for detecting the fault.

The fault detection component may be configured for deactivating the at least one aerosolization system.

The fault detection component may be configured for operating based on a temperature.

The fault detection component may be configured for performing the deactivating upon detection of the fault.

The fault detection component may comprise at least one of a positive temperature coefficient element and a negative temperature coefficient element.

The replacement part may be configured for operating according to an operation mode depending on the voltage provided by the power source.

The voltage control component may be configured for sensing the voltage provided by the power source.

The voltage control component may be configured for deactivating the aerosolization system, if the voltage provided by the power source is below a lower threshold.

The replacement part may comprise the data storage component.

The data storage component may be configured for storing identification data.

The data storage component may be configured for storing state data.

The data storage component may be configured for sending at least one of identification data and state data.

The data storage component may be configured for sending the data to the base of the mobile inhaler as disclosed above.

The data storage component may be configured for sending the data to a data transmission component the base of the mobile inhaler as discussed above.

The data storage component may be configured for storing identification data that match an identity-criterion.

The replacement part may be configured for being activated by the base of the mobile inhaler.

The replacement part may be configured for outputting the indicator for being activated, preferably for being activated by the base of the mobile inhaler. As discussed above, the outputting may also be performed by forwarding the indicator to another device, such as a smart phone, which may then be configured for outputting the indicator.

The replacement part may be configured for receiving data.

The replacement part may be configured for receiving the data from the remainder of the mobile inhaler and/or the base of the mobile inhaler.

The data storage component may be configured for receiving the data.

The replacement part, preferably the data storage component, may be configured for receiving the data relating to the usage of the mobile inhaler and/or the base of the mobile inhaler with which it is in use.

The replacement part may be configured for receiving the data relating to an amount of dispensed substance from the at least one reservoir of the replacement part.

The replacement part may be configured for receiving the data relating to the use of the aerosolization system of the replacement part.

The replacement part may be configured for receiving state data from the remainder of the mobile inhaler and/or the base of the mobile inhaler.

The replacement part, preferably the data storage component, may be configured for receiving data configured for deactivating the replacement part.

The replacement part, preferably the data storage component, may be configured for receiving data configured for instructing at least one the remainder of the mobile inhaler and the base of the mobile inhaler to not activate the replacement part.

The data storage component may be configured for data exchange by the wireless communication, such as the connection by electro-magnetic radiation.

The data storage component may comprise the identification unit.

The replacement part may comprise the housing. The data storage component may comprise the adhesive layer fixing the identification unit to the housing of the replacement part.

The data storage component may comprise the surface layer. The surface layer may be configured for covering the identification unit. The surface layer may be substantially made from the polymer or the polymer composition.

The data storage component may comprise the adhesive layer which fixes the surface layer at least partially on the identification unit.

The data storage component may be configured for data exchange by an electrical connection.

The replacement part may comprise the contacts, as discussed above. The data storage component may be configured for data exchange by means of the contacts.

The identification unit may be permanently attached to the replacement part.

The identification unit may be configured to be destroyed when removed from the replacement part.

The at least one aerosolization system may be at least two aerosolization systems, each configured for aerosolizing a different liquid.

The at least one reservoir may be at least two reservoirs. Each reservoir may be configured for holding a different liquid to be aerosolized. Each aerosolization system may comprise at least one of the at least two reservoirs. For example, each aerosolization system may comprise one of the at least two reservoirs.

One of the liquids may comprises at least one substance selected from nicotine and the active pharmaceutical ingredient, and one of the liquids may not comprise said substance.

One of the aerosolization systems may comprise a heating unit and may be configured for thermal aerosolization. The other may be configured for nebulization.

The data storage component may be configured for storing identification data for at least one of each of the aerosolization systems and each of the reservoirs, that is, for each of the aerosolization systems, each of the reservoirs, or each of the reservoirs and each of the aerosolization systems.

The data storage component may be configured for storing state data for at least one of each of the aerosolization systems and each of the reservoirs.

Each of the aerosolization systems may be configured for being independently activated by the base of the mobile inhaler.

The replacement part may be configured for the outputting of the indicator of the different aerosolization systems being activated.

The replacement part, preferably the data storage component, may be configured for receiving the data relating to an amount of dispensed substance from each of the two reservoirs of the replacement part.

The replacement part, preferably the data storage component, may be configured for receiving the data relating to the use of each of the aerosolization systems of the replacement part.

The data storage component may be configured for receiving state data for at least one of each of the aerosolization systems and each of the reservoirs.

The replacement part may be configured for receiving state data for at least one of each of the aerosolization systems and each of the reservoirs from the remainder of the mobile inhaler and/or the base of the mobile inhaler.

In a fourth embodiment, computer program products are disclosed.

A first computer program product may comprise instructions, which instructions cause the mobile inhaler to perform the steps for which the mobile inhaler and/or its components are configured according to the above disclosure.

A second computer program product may comprise instructions, which instructions cause the base of the mobile inhaler to perform the steps for which the base of the mobile inhaler and/or its components are configured.

A third computer program product may comprise instructions causing the data storage component to perform the steps for which it is configured.

The following embodiments also form part of the invention.

Below, system embodiments will be discussed. These embodiments are abbreviated by the letter "S" followed by a number. Whenever reference is herein made to "system embodiments", these embodiments are meant.
S1. A mobile inhaler (10), comprising a power source (30) and at least one aerosolization system (20).
S2. The mobile inhaler (10) according to the preceding embodiment, further comprising an aerosolization system control component (40).
S3. The mobile inhaler (10) according to the preceding embodiment, wherein the aerosolization system control component comprises an electronic control element.
S4. The mobile inhaler (10) according to the preceding embodiment, wherein the electronic control element comprises a transistor, such as a MOS transistor.
S5. The mobile inhaler (10) according to any of the preceding embodiments, wherein the mobile inhaler (10) comprises at least one reservoir, wherein each reservoir is configured for holding a liquid to be aerosolized.
S6. The mobile inhaler (10) according to the preceding embodiment, wherein each aerosolization system comprises at least one reservoir.
S7. The mobile inhaler (10) according to any of the preceding embodiments, wherein the mobile inhaler (10) is configured for aerosolizing at least one liquid comprising at least one of nicotine and an active pharmaceutical ingredient.
S8. The mobile inhaler (10) according to any of the preceding embodiments, wherein the mobile inhaler further comprises a control component (50).
S9. The mobile inhaler according to any of the preceding embodiments, wherein the mobile inhaler further comprises a voltage control component (34).
S10. The mobile inhaler according to any of the preceding embodiments, wherein the mobile inhaler comprises a data transmission component (52).
S11. The mobile inhaler according to any of the preceding embodiments, wherein the at least one aerosolization system (20) comprises at least one vaporization system, and wherein the at least one vaporization system is configured for heating of the at least one liquid to be aerosolized.
S12. The mobile inhaler according to any of the preceding embodiments, wherein each aerosolization system (20) is a vaporization system.
S13. The mobile inhaler according to the preceding embodiment, wherein the at least one vaporization system is configured for heating of the liquid to be aerosolized.
S14. The mobile inhaler according to any of the three preceding embodiments, wherein each vaporization system is configured for thermal aerosolization.
S15. The mobile inhaler according to any of the four preceding embodiments, wherein each vaporization system comprises a heating unit, such as a heating coil or a ceramic heating element.
S16. The mobile inhaler according to any of the preceding embodiments, wherein each aerosolization system (20) comprises a connection to the power source (30).
S17. The mobile inhaler according to any of the preceding embodiments with the features of S2, wherein the mobile inhaler comprises a fault detection component.
S18. The mobile inhaler according to the preceding embodiment, wherein the fault detection component is configured for detecting a fault.
S19. The mobile inhaler according to any of the two preceding embodiments, wherein the fault detection component is configured for deactivating the at least one aerosolization system.
S20. The mobile inhaler according to any of the three preceding embodiments, wherein the fault detection component is configured for deactivating at least a part of the aerosolization control system.
S21. The mobile inhaler according to any of the four preceding embodiments, wherein the fault detection component is configured for deactivating the power source.
S22. The mobile inhaler according to any of the five preceding embodiments, wherein the fault detection component is configured for operating based on a temperature.
S23. The mobile inhaler according to the preceding embodiment, wherein the fault detection component is configured for operating based on a temperature of the power source.
S24. The mobile inhaler according to any of the two preceding embodiments, wherein the fault detection component is configured for operating based on a temperature of a housing of the mobile inhaler.
S25. The mobile inhaler according to any of the preceding embodiments with the features of at least one of S19, S20 and S21, wherein the fault detection component is configured for performing the deactivating upon detection of the fault.
S26. The mobile inhaler according to any of the preceding embodiments with the features of S22, wherein the fault detection component comprises at least one of a positive temperature coefficient element and a negative temperature coefficient element.
S27. The mobile inhaler according to any of the preceding embodiments, wherein the mobile inhaler is configured for operating according to an operation mode depending on a voltage provided by the power source.
S28. The mobile inhaler according to the preceding embodiment and with the features of S9, wherein the voltage control component is configured for sensing a voltage provided by the power source.
S29. The mobile inhaler according to the preceding embodiment, wherein the voltage control component is configured for deactivating the aerosolization system, if the voltage provided by the power source is below a lower threshold.
S30. The mobile inhaler according to any of the two preceding embodiments, wherein the voltage control component is configured for reducing a voltage provided to the at least one aerosolization system, if the voltage provided by the power source is above an upper threshold.
S31. The mobile inhaler according to any of the three preceding embodiments, wherein mobile inhaler is configured for substantially providing the voltage provided by the power source to the aerosolization system, if the voltage provided by the power source is between the lower and the upper threshold.
S32. The mobile inhaler according to any of the preceding embodiments with the features of S9, wherein the voltage control component is configured for reducing the voltage provided to the aerosolization system by means of the aerosolization system control component.
S33. The mobile inhaler according to the preceding embodiment and with the features of S18, wherein the voltage control component is configured for reducing the voltage provided to the aerosolization system and/or the aerosolization system control component if the fault detection component detects a fault.
S34. The mobile inhaler according to any of the two preceding embodiments and with the features of at least one of S19, S20 and S21, wherein the fault detection component is configured for performing the deactivating by means of the voltage control component. S35. The mobile inhaler according to any of the preceding embodiments with the features of S9, wherein the voltage control component comprises at least one transistor, and preferably a power converter, such as a step-down converter.
S36. The mobile inhaler according to any of the preceding embodiments, wherein the mobile inhaler comprises a replacement part.
S37. The mobile inhaler according to the preceding embodiment, wherein the replacement part comprises the at least one aerosolization system.
S38. The mobile inhaler according to any of the two preceding embodiments and with the features of S5, wherein the replacement part comprises at least one of the at least one reservoir.
S39. The mobile inhaler according to any of the three preceding embodiments, wherein the replacement part comprises a data storage component (24).
S40. The mobile inhaler according to the preceding embodiment, wherein the data storage component (24) is configured for storing identification data.
S41. The mobile inhaler according to any of the two preceding embodiments, wherein the data storage component (24) is configured for storing state data.
S42. The mobile inhaler according to any of the preceding embodiments with the features of S36 and S10, wherein the data transmission component is configured for receiving at least one of identification data and state data from the replacement part.
S43. The mobile inhaler according to the preceding embodiment and with the features of S39, wherein the data transmission component is configured for receiving at least one of identification data and state data from the data storage component (24).
S44. The mobile inhaler according to any of the preceding two embodiments, wherein the mobile inhaler is configured for activating the replacement part only if the identification data match an identity-criterion.
S45. The mobile inhaler according to any of the preceding three embodiments, wherein the mobile inhaler is configured for activating the replacement part only if the state data match a state-criterion.
S46. The mobile inhaler according to any of the two preceding embodiments, wherein the mobile inhaler is configured for outputting an indicator of at least one of the matching of the state-criterion and the identity-criterion.
S47. The mobile inhaler according to any of the three preceding embodiments, wherein the mobile inhaler is configured for receiving at least one of the identity-criterion and the state-criterion from a server.
S48. The mobile inhaler according to any of the preceding embodiments and with the features of S36 and S10, wherein the data transmission component is configured for sending data to the replacement part.
S49. The mobile inhaler according to the preceding embodiment and with the features of S39, wherein the data transmission component is configured for sending data to the data storage component.
S50. The mobile inhaler according to any of the two preceding embodiments, wherein the data transmission component is configured for sending data relating to a usage of the mobile inhaler to the replacement part, preferably to the data storage component.
S51. The mobile inhaler according to any of the three preceding embodiments and with the features of S38, wherein the data transmission component is configured for sending data relating to an amount of dispensed substance from the at least one reservoir of the replacement part.
S52. The mobile inhaler according to any of the four preceding embodiments and with the features of S37, wherein the data transmission component is configured for sending data relating to a use of the at least one aerosolization system of the replacement part.
S53. The mobile inhaler according to any of the five preceding embodiments with the features of S41, wherein the data transmission component is configured for sending state data to the replacement part.
S54. The mobile inhaler according to the preceding embodiment and with the features of S45, wherein the data transmission component is configured for sending state data to the replacement part which do not match the state-criterion based on the data relating to the amount of dispensed substance.
S55. The mobile inhaler according to any of the two preceding embodiments and with the features of S45, wherein the data transmission component is configured for sending state data to the replacement part which do not match the state-criterion based on the data relating to the use of the aerosolization system.
S56. The mobile inhaler according to any of the preceding embodiments with the features of S53, S45 and S19, wherein the data transmission component is configured for sending state data to the replacement part which do not match the state-criterion if the fault detection component deactivates the aerosolization system.
S57. The mobile inhaler according to any of the preceding embodiments with the features of S39 and S10, wherein the data storage component and the data transmission component are configured for data exchange by a wireless communication, such as a connection by electro-magnetic radiation.
S58. The mobile inhaler according to the preceding embodiment, wherein the data storage component comprises an identification unit (70).
S59. The mobile inhaler according to the preceding embodiment, wherein the replacement part comprises a housing (78), and wherein the data storage component comprises an adhesive layer (72) fixing the identification unit (70) to the housing (78) of the replacement part.
S60. The mobile inhaler according to any of the three preceding embodiments, wherein the data storage component comprises a surface layer (78), wherein the surface layer (78) is configured for covering the identification unit (70).
S61. The mobile inhaler according to any of the preceding embodiments with the features of S58 and S60, wherein the data storage component comprises an adhesive layer (74) fixing the surface layer (78) at least partially on the identification unit (70).
S62. The mobile inhaler according to any of the preceding embodiments with the features of S39 and S10, wherein the data storage component and the data transmission component are configured for data exchange by an electrical connection.
S63. The mobile inhaler according to the preceding embodiment, wherein the replacement part comprises contacts (26), and wherein the data storage component and the data transmission component are configured for data exchange by means of the contacts (26).
S64. The mobile inhaler according to any of the preceding embodiments with the features of S58, wherein the identification unit is permanently attached to the replacement part.
S65. The mobile inhaler according to any of the preceding embodiments with the features of S58, wherein the identification unit is configured to be destroyed when removed from the replacement part.
S66. The mobile inhaler according to any of the preceding embodiments, wherein the at least one aerosolization system is at least two aerosolization systems, each configured for aerosolizing a different liquid.
S67. The mobile inhaler according to the preceding embodiment and with the features of S5, wherein the at least one reservoir is at least two reservoirs, wherein each reservoir is configured for holding a different liquid to be aerosolized.
S68. The mobile inhaler according to any of the two preceding embodiments and with the features of S2, wherein the mobile inhaler comprises a second aerosolization system control component (140).
S69. The mobile inhaler according to any of the three preceding embodiments, wherein one of the liquids comprises at least one substance selected from nicotine and the active pharmaceutical ingredient, and one of the liquids does not comprise said substance.
S70. The mobile inhaler according to any of the preceding embodiments with the features of S66, wherein one of the aerosolization systems comprises a heating unit and is configured for thermal aerosolization, and the other is configured for nebulization.
S71. The mobile inhaler according to any of the preceding embodiments with the features of S66 and S19, wherein the fault detection component is configured for deactivating the at least two aerosolization systems.
S72. The mobile inhaler according to any of the preceding embodiments with the features of S68 and S20, wherein the fault detection component is configured for deactivating at least a part of the aerosolization control system (40) and of the second aerosolization control system.
S73. The mobile inhaler according to any of the preceding embodiments with the features of S66 and S37, wherein the replacement part comprises the at least two aerosolization systems.
S74. The mobile inhaler according to any of the preceding embodiments with the features of S67 and S38, wherein the replacement part comprises the at least two reservoirs.
S75. The mobile inhaler according to any of the two preceding embodiments and with the features of S40, wherein the data storage component (24) is configured for storing identification data for at least one of each of the aerosolization systems and each of the reservoirs.
S76. The mobile inhaler according to any of the three preceding embodiments and with the features of S41, wherein the data storage component (24) is configured for storing state data for at least one of each of the aerosolization systems and each of the reservoirs.
S77. The mobile inhaler according to any of the preceding embodiments with the features of S75, wherein the mobile inhaler is configured for activating each aerosolization system with the corresponding reservoir only if the corresponding identification data match the identification-criterion.
S78. The mobile inhaler according to any of the preceding embodiments with the features of S76, wherein the mobile inhaler is configured for activating each aerosolization system with the corresponding reservoir only if the corresponding state data match the state-criterion.
S79. The mobile inhaler according to any of the preceding embodiments with the features of S51, wherein the data transmission component is configured for sending data relating to an amount of dispensed substance from each of the two reservoirs of the replacement part.
S80. The mobile inhaler according to any of the four preceding embodiments and with the features of S73 and S52, wherein the data transmission component is configured for sending data relating to the use of each of the aerosolization systems of the replacement part.
S81. The mobile inhaler according to any of the preceding embodiments with the features of S53, wherein the data transmission component is configured for sending the state data for each of the at least two aerosolization systems and each of the reservoirs.
S82. The mobile inhaler according to any of the preceding embodiments with the features of S75, wherein the mobile inhaler is configured for activating one aerosolization system with the corresponding reservoir only if the corresponding identification data match the identification-criterion, and the other aerosolization system with the corresponding reservoir only if the identification data corresponding to both aerosolization systems with the corresponding reservoirs match the identification-criterion.
S83. The mobile inhaler according to any of the preceding embodiments with the features of S76, wherein the mobile inhaler is configured for activating one aerosolization system with the corresponding reservoir only if the corresponding state data match the state-criterion, and the other aerosolization system with the corresponding reservoir only if the state data corresponding to both aerosolization systems with the corresponding reservoirs match the state-criterion.
S84. The mobile inhaler according to any of the preceding embodiments with the features of S75, wherein the mobile inhaler is configured for activating each aerosolization system with the corresponding reservoir only if the identification data corresponding to the at least two aerosolization systems match the identification-criterion.
S85. The mobile inhaler according to any of the preceding embodiments with the features of S76, wherein the mobile inhaler is configured for activating each aerosolization system with the corresponding reservoir only if the state data corresponding to the at least two aerosolization systems match the state-criterion.

Below, embodiments of a base of a mobile inhaler will be discussed. These embodiments are abbreviated by the letter "M" followed by a number. Whenever reference is herein made to "inhaler base embodiments", these embodiments are meant.
M1. A base of a mobile inhaler (10), comprising a power source (30).
M2. The base according to the preceding embodiment, wherein the base of the mobile inhaler comprises at least one aerosolization system (20).
M3. The base according to any of the two preceding embodiments, wherein the base of the mobile inhaler is configured to be connected to the at least one aerosolization system (20).
M4. The base according to any of the three preceding embodiments, wherein the base of the mobile inhaler further comprises an aerosolization system control component (40).
M5. The base according to the preceding embodiment, wherein the aerosolization system control component comprises an electronic control element.
M6. The base according to the preceding embodiment, wherein the electronic control element comprises a transistor, such as a MOS transistor.
M7. The base according to any of the preceding inhaler base embodiments, wherein the base of the mobile inhaler comprises at least one reservoir, wherein the reservoir is configured for holding a liquid to be aerosolized.
M8. The base according to any of the preceding inhaler base embodiments, wherein the base of the mobile inhaler is configured to be connected to the at least one reservoir, wherein each reservoir is configured for holding the liquid to be aerosolized.
M9. The base according to the preceding embodiment, wherein each aerosolization system comprises at least one reservoir.
M10. The base according to any of the preceding inhaler base embodiments, wherein the base of the mobile inhaler is configured for aerosolizing a liquid comprising at least one of nicotine and an active pharmaceutical ingredient.
M11. The base according to any of the preceding inhaler base embodiments, wherein the base of the mobile inhaler further comprises a control component (50).
M12. The base according to any of the preceding inhaler base embodiments, wherein the base of the mobile inhaler further comprises a voltage control component (34).
M13. The base according to any of the preceding inhaler base embodiments, wherein the base of the mobile inhaler comprises a data transmission component (52).
M14. The base according to any of the preceding inhaler base embodiments with the features of M2, wherein the at least one aerosolization system (20) comprises at least one vaporization system, and wherein the at least one vaporization system is configured for heating of the liquid to be aerosolized.
M15. The base according to any of the preceding inhaler base embodiments, wherein each aerosolization system (20) is a vaporization system.
M16. The base according to the preceding embodiment, wherein the at least one vaporization system is configured for heating of the liquid to be aerosolized.
M17. The base according to any of the three preceding embodiments, wherein each vaporization system is configured for thermal aerosolization.
M18. The base according to any of the four preceding embodiments, wherein each vaporization system comprises a heating unit, such as a heating coil or a ceramic heating element.
M19. The base according to any of the preceding inhaler base embodiments with the features of M2, wherein each aerosolization system (20) comprises a connection to the power source (30).
M20. The base according to any of the preceding inhaler base embodiments with the features of M4, wherein the base of the mobile inhaler comprises a fault detection component.
M21. The base according to the preceding embodiment, wherein the fault detection component is configured for detecting a fault.
M22. The base according to any of the two preceding embodiments, wherein the fault detection component is configured for deactivating the aerosolization system.
M23. The base according to any of the three preceding embodiments, wherein the fault detection component is configured for deactivating at least a part of the aerosolization control system.
M24. The base according to any of the four preceding embodiments, wherein the fault detection component is configured for deactivating the power source.
M25. The base according to any of the five preceding embodiments, wherein the fault detection component is configured for operating based on a temperature.
M26. The base according to the preceding embodiment, wherein the fault detection component is configured for operating based on a temperature of the power source.
M27. The base according to any of the two preceding embodiments, wherein the fault detection component is configured for operating based on a temperature of a housing of the base of the mobile inhaler.
M28. The base according to any of the preceding embodiments with the features of at least one of M22, M23 and M24, wherein the fault detection component is configured for performing the deactivating upon detection of the fault.
M29. The base according to any of the preceding embodiments with the features of M25, wherein the fault detection component comprises at least one of a positive temperature coefficient element and a negative temperature coefficient element.
M30. The base according to any of the preceding inhaler base embodiments, wherein the base is configured for operating according to an operation mode depending on a voltage provided by the power source.
M31. The base according to the preceding embodiment and with the features of M12, wherein the voltage control component is configured for sensing a voltage provided by the power source.
M32. The base according to the preceding embodiment, wherein the voltage control component is configured for deactivating the aerosolization system, if the voltage provided by the power source is below a lower threshold.
M33. The base according to any of the two preceding embodiments, wherein the voltage control component is configured for reducing a voltage provided to the at least one aerosolization system, if the voltage provided by the power source is above an upper threshold.
M34. The base according to any of the three preceding embodiments, wherein the base of the mobile inhaler is configured for substantially providing the voltage provided by the power source to the aerosolization system, if the voltage provided by the power source is between the lower and the upper threshold.
M35. The base according to any of the preceding embodiments with the features of M12, wherein the voltage control component is configured for reducing the voltage provided to the aerosolization system by means of the aerosolization system control component.
M36. The base according to the preceding embodiment and with the features of M21, wherein the voltage control component is configured for reducing the voltage provided to the aerosolization system and/or the aerosolization system control component if the fault detection component detects a fault.
M37. The base according to any of the two preceding embodiments and with the features of at least one of M22, M23 and M24, wherein the fault detection component is configured for performing the deactivating by means of the voltage control component.
M38. The base according to any of the preceding inhaler base embodiments with the features of M12, wherein the voltage control component comprises at least one transistor, and preferably a power converter, such as a step-down converter.
M39. The base according to any of the preceding embodiments, wherein the base of the mobile inhaler is configured to be connected to a replacement part.
M40. The base according to the preceding embodiment, wherein the replacement part comprises the at least one aerosolization system.
M41. The base according to any of the two preceding embodiments and with the features of M8, wherein the replacement part comprises at least one of the at least one reservoir.
M42. The base according to any of the three preceding embodiments, wherein the replacement part comprises an data storage component (24).
M43. The base according to the preceding embodiment, wherein the data storage component (24) is configured for storing identification data.
M44. The base according to any of the two preceding embodiments, wherein the data storage component (24) is configured for storing state data.
M45. The base according to any of the preceding inhaler base embodiments with the features of M39 and M13, wherein the data transmission component is configured for receiving at least one of identification data and state data from the replacement part.
M46. The base according to the preceding embodiment and with the features of M42, wherein the data transmission component is configured for receiving at least one of identification data and state data from the data storage component (24).
M47. The base according to any of the preceding two embodiments, wherein the base of the mobile inhaler is configured for activating the replacement part only if the identification data match an identity-criterion.
M48. The base according to any of the preceding three embodiments, wherein the base of the mobile inhaler is configured for activating the replacement part only if the state data match a state-criterion.
M49. The base according to any of the two preceding embodiments, wherein the base of the mobile inhaler is configured for outputting an indicator of at least one of the matching of the state-criterion and the identity-criterion.
M50. The base according to any of the three preceding embodiments, wherein the base of the mobile inhaler is configured for receiving at least one of the identity-criterion and the state-criterion from a server.
M51. The base according to any of the preceding inhaler base embodiments and with the features of M39 and M13, wherein the data transmission component is configured for sending data to the replacement part.
M52. The base according to the preceding embodiment and with the features of M42, wherein the data transmission component is configured for sending data to the data storage component.
M53. The base according to any of the two preceding embodiments, wherein the data transmission component is configured for sending data relating to a usage of the mobile inhaler to the replacement part, preferably to the data storage component.
M54. The base according to any of the three preceding embodiments and with the features of M41, wherein the data transmission component is configured for sending data relating to an amount of dispensed substance from the at least one reservoir of the replacement part.
M55. The base according to any of the four preceding embodiments and with the features of M40, wherein the data transmission component is configured for sending data relating to a use of the aerosolization system of the replacement part.
M56. The base according to any of the five preceding embodiments and with the features of M44, wherein the data transmission component is configured for sending state data to the replacement part.
M57. The base according to the preceding embodiment and with the features of M48, wherein the data transmission component is configured for sending state data to the replacement part which do not match the state-criterion based on the data relating to the amount of dispensed substance.
M58. The base according to any of the two preceding embodiments and with the features of M48, wherein the data transmission component is configured for sending state data to the replacement part which do not match the state-criterion based on the data relating to the use of the aerosolization system.
M59. The base according to any of the preceding inhaler base embodiments with the features of M56, M48 and M22, wherein the data transmission component is configured for sending state data to the replacement part which do not match the state-criterion if the fault detection component deactivates the aerosolization system.
M60. The base according to any of the preceding inhaler base embodiments with the features of M42 and M13, wherein the data transmission component is configured for data exchange with the data storage component by a wireless communication, such as a connection by electro-magnetic radiation.
M61. The base according to the preceding embodiment, wherein the data storage component comprises an identification unit (70).
M62. The base according to any of the two preceding embodiments, wherein the data transmission component comprises an antenna.
M63. The base according to any of the preceding inhaler base embodiments with the features of M42 and M13, wherein the data transmission component is configured for data exchange with the data storage component by an electrical connection.
M64. The base according to the preceding embodiment, wherein the base of the mobile inhaler comprises contacts (26), and wherein the data transmission component is configured for data exchange with the data storage component by means of the contacts (26).
M65. The mobile inhaler according to any of the preceding inhaler base embodiments with the features of M61, wherein the identification unit is permanently attached to the replacement part.
M66. The base according to any of the preceding inhaler base embodiments with the features of at least one of M2 and M3, wherein the at least one aerosolization system is at least two aerosolization systems, each configured for aerosolizing a different liquid.
M67. The base according to the preceding embodiment and with the features of at least one of M7 and M8, wherein the at least one reservoir are at least two reservoirs, wherein each reservoir is configured for holding a different liquid to be aerosolized.
M68. The base according to any of the two preceding embodiments and with the features of M4, wherein the base of the mobile inhaler comprises a second aerosolization system control component (140).
M69. The base according to any of the three preceding embodiments, wherein one of the liquids comprises at least one substance selected from nicotine and the active pharmaceutical ingredient, and one of the liquids does not comprise said substance.
M70. The base according to any of the preceding embodiments with the features of M66, wherein one of the aerosolization systems comprises a heating unit and is configured for thermal aerosolization, and the other is configured for nebulization.
M71. The base according to any of the preceding embodiments with the features of M66 and M22, wherein the fault detection component is configured for deactivating the at least two aerosolization systems.
M72. The base according to any of the preceding embodiments with the features of M68 and M23, wherein the fault detection component is configured for deactivating at least a part of the aerosolization control system (40) and of the second aerosolization control system.
M73. The base according to any of the preceding embodiments with the features of M66 and M40, wherein the replacement part comprises the at least two aerosolization systems.
M74. The base according to any of the preceding inhaler base embodiments with the features of M67 and M41, wherein the replacement part comprises the at least two reservoirs.
M75. The base according to any of the two preceding embodiments and with the features of M43, wherein the data storage component (24) is configured for storing identification data for at least one of each of the aerosolization systems and each of the reservoirs.
M76. The base according to any of the three preceding embodiments and with the features of M44, wherein the data storage component (24) is configured for storing state data for at least one of each of the aerosolization systems and each of the reservoirs.
M77. The base according to any of the preceding inhaler base embodiments with the features of M75, wherein the base of the mobile inhaler is configured for activating each aerosolization system with the corresponding reservoir only if the corresponding identification data from the data storage component of the replacement part match the identification-criterion.
M78. The base according to any of the preceding inhaler base embodiments with the features of M76, wherein the base of the mobile inhaler is configured for activating each aerosolization system with the corresponding reservoir only if the corresponding state data match the state-criterion.
M79. The base according to any of the preceding inhaler base embodiments with the features of M54, wherein the data transmission component is configured for sending data relating to an amount of dispensed substance from each of the two reservoirs of the replacement part.
M80. The base according to any of the four preceding embodiments and with the features of M73 and M55, wherein the data transmission component is configured for sending data relating to the use of each of the aerosolization systems of the replacement part.
M81. The base according to any of the preceding embodiments with the features of M56, wherein the data transmission component is configured for sending the state data for each of the at least one aerosolization systems and each of the reservoirs.
M82. The base according to any of the preceding embodiments with the features of M75, wherein the base is configured for activating one aerosolization system with the corresponding reservoir only if the corresponding identification data match the identification-criterion, and the other aerosolization system with the corresponding reservoir only if the identification data corresponding to both aerosolization systems with the corresponding reservoirs match the identification-criterion.
M83. The base according to any of the preceding embodiments with the features of M76, wherein the base is configured for activating one aerosolization system with the corresponding reservoir only if the corresponding state data match the state-criterion, and the other aerosolization system with the corresponding reservoir only if the state data corresponding to both aerosolization systems with the corresponding reservoirs match the state-criterion.
M84. The base according to any of the preceding embodiments with the features of M75, wherein the base is configured for activating each aerosolization system with the corresponding reservoir only if the identification data corresponding to the at least two aerosolization systems match the identification-criterion.
M85. The base according to any of the preceding embodiments with the features of M76, wherein the base is configured for activating each aerosolization system with the corresponding reservoir only if the state data corresponding to the at least two aerosolization systems match the state-criterion.

Below, embodiments of a replacement part for a mobile will be discussed. These embodiments are abbreviated by the letter "R" followed by a number. Whenever reference is herein made to "replacement part embodiments", these embodiments are meant.
R1. A replacement part, comprising at least one aerosolization system (20).
R2. The replacement part according to the preceding embodiment, wherein the replacement part is configured for use with a base of a mobile inhaler according to any of the inhaler base embodiments.
R3. The replacement part according to any of the preceding replacement part embodiments, wherein each aerosolization system comprises at least one reservoir.
R4. The replacement part according to the preceding embodiment, wherein each reservoir is configured for holding a liquid to be aerosolized.
R5. The replacement part according to any of the preceding replacement part embodiments, wherein at least one of the at least one aerosolization system is configured for aerosolizing a liquid comprising at least one of nicotine and an active pharmaceutical ingredient.
R6. The replacement part according to any of the preceding replacement part embodiments, wherein the replacement part further comprises a voltage control component (34).
R7. The replacement part according to any of the preceding replacement part embodiments, wherein the replacement part is a replacement part for a mobile inhaler according to any of the system embodiments.
R8. The replacement part according to any of the preceding replacement part embodiments, wherein the replacement part is a replacement part for regular replacement.
R9. The replacement part according to any of the preceding replacement part embodiments, wherein the replacement part is a replacement part for replacement by an end user.
R10. The replacement part according to any of the preceding replacement part embodiments, wherein the at least one aerosolization system (20) comprises at least one vaporization system, and wherein the at least one vaporization system is configured for heating of the liquid to be aerosolized.
R11. The replacement part according to any of the preceding replacement part embodiments, wherein each aerosolization system (20) is a vaporization system, and wherein each vaporization system is configured for heating of the liquid to be aerosolized.
R12. The replacement part according to the preceding embodiment, wherein the at least one vaporization system is configured for heating of the liquid to be aerosolized.
R13. The replacement part according to any of the three preceding embodiments, wherein each vaporization system is configured for thermal aerosolization.
R14. The replacement part according to any of the four preceding embodiments, wherein each vaporization system comprises a heating unit, such as a heating coil or a ceramic heating element.
R15. The replacement part according to any of the preceding replacement part embodiments, wherein each aerosolization system (20) is configured to be connected to a power source (30).
R16. The replacement part according to any of the preceding replacement part embodiments, wherein the replacement part comprises a fault detection component.
R17. The replacement part according to the preceding embodiment, wherein the fault detection component is configured for detecting a fault.
R18. The replacement part according to any of the two preceding embodiments, wherein the fault detection component is configured for deactivating the at least one aerosolization system.
R19. The replacement part according to any of the three preceding embodiments, wherein the fault detection component is configured for operating based on a temperature.
R20. The replacement part according to any of the preceding replacement part embodiments with the features of R18, wherein the fault detection component is configured for performing the deactivating upon detection of the fault.
R21. The replacement part according to any of the preceding embodiments with the features of R19, wherein the fault detection component comprises at least one of a positive temperature coefficient element and a negative temperature coefficient element.
R22. The replacement part according to any of the preceding replacement part embodiments, wherein the replacement part is configured for operating according to an operation mode depending on a voltage provided by the power source.
R23. The replacement part according to the preceding embodiment and with the features of R6, wherein the voltage control component is configured for sensing the voltage provided by the power source.
R24. The replacement part according to the preceding embodiment, wherein the voltage control component is configured for deactivating the aerosolization system, if the voltage provided by the power source is below a lower threshold.
R25. The replacement part according to any of the preceding replacement part embodiments, wherein the replacement part comprises an data storage component (24).
R26. The replacement part according to the preceding embodiment, wherein the data storage component (24) is configured for storing identification data.
R27. The replacement part according to any of the two preceding embodiments, wherein the data storage component (24) is configured for storing state data.
R28. The replacement part according to any of the preceding replacement part embodiments with the features of R25, wherein the data storage component is configured for sending at least one of identification data and state data.
R29. The replacement part according to the preceding embodiment, wherein the data storage component is configured for sending the data to a base of a mobile inhaler according to any of the inhaler base embodiments.
R30. The replacement part according to any of the two preceding embodiments and with the features of R25, wherein the data storage component is configured for sending the data to a data transmission component of a base of a mobile inhaler according to any of the inhaler base embodiments with the features of M13.
R31. The replacement part according to any of the preceding three embodiments, wherein the data storage component is configured for storing identification data that match an identity-criterion.
R32. The replacement part according to any of the four preceding embodiments and with the features of R2, wherein the replacement part is configured for being activated by the base of the mobile inhaler, wherein the base of the mobile inhaler is preferably according to at least one of M22, M32, M47 and M48 or any of their depending embodiments.
R33. The replacement part according to the preceding embodiment, wherein the replacement part is configured for outputting an indicator for being activated, preferably for being activated by the base of the mobile inhaler.
R34. The replacement part according to any of the preceding replacement part embodiments, wherein the replacement part is configured for receiving data.
R35. The replacement part according to the preceding embodiment and with the features of at least one of R2 and R7, wherein the replacement part is configured for receiving the data from a remainder of the mobile inhaler and/or the base of the mobile inhaler.
R36. The replacement part according to any of the two preceding embodiments and with the features of R25, wherein the data storage component is configured for receiving the data.
R37. The replacement part according to any of the three preceding embodiments and with the features of at least one of R2 and R7, wherein the replacement part, preferably the data storage component, is configured for receiving data relating to a usage of the mobile inhaler and/or the base of the mobile inhaler with which it is in use.
R38. The replacement part according to any of the four preceding embodiments, wherein the replacement part is configured for receiving data relating to an amount of dispensed substance from the at least one reservoir of the replacement part.
R39. The replacement part according to any of the five preceding embodiments, wherein the replacement part is configured for receiving data relating to a use of the aerosolization system of the replacement part.
R40. The replacement part according to any of the six preceding embodiments with the features of R27 and at least one of R2 and R7, wherein the replacement part is configured receiving state data from the remainder of the mobile inhaler and/or the base of the mobile inhaler.
R41. The replacement part according to any of the preceding replacement part embodiments with the features of R35, preferably with R36, wherein the replacement part, preferably the data storage component, is configured for receiving data configured for deactivating the replacement part.
R42. The replacement part according to any of the preceding replacement part embodiments with the features of R35, preferably with R36, wherein the replacement part, preferably the data storage component, is configured for receiving data configured for instructing at least one the remainder of the mobile inhaler and the base of the mobile inhaler to not activate the replacement part.
R43. The replacement part according to any of the preceding replacement part embodiments with the features of R25, wherein the data storage component is configured for data exchange by a wireless communication, such as a connection by electro-magnetic radiation.
R44. The replacement part according to the preceding embodiment, wherein the data storage component comprises an identification unit (70).
R45. The replacement part according to the preceding embodiment, wherein the replacement part comprises a housing (78), and wherein the data storage component comprises an adhesive layer (72) fixing the identification unit (70) to the housing (78) of the replacement part.
R46. The replacement part according to any of the three preceding embodiments, wherein the data storage component comprises a surface layer (78), wherein the surface layer (78) is configured for covering the identification unit (70).
R47. The replacement part according to any of the preceding embodiments with the features of R44 and R46, wherein the data storage component comprises an adhesive layer (74) fixing the surface layer (78) at least partially on the identification unit (70).
R48. The replacement part according to any of the preceding replacement part embodiments with the features of R25, wherein the data storage component is configured for data exchange by an electrical connection.
R49. The replacement part according to the preceding embodiment, wherein the replacement part comprises contacts (26), and wherein the data storage component is configured for data exchange by means of the contacts (26).
R50. The replacement part according to any of the preceding replacement part embodiments with the features of R44, wherein the identification unit is permanently attached to the replacement part.
R51. The replacement part according to any of the preceding replacement part embodiments with the features of R44, wherein the identification unit is configured to be destroyed when removed from the replacement part.
R52. The replacement part according to any of the preceding replacement part embodiments, wherein the at least one aerosolization system is at least two aerosolization systems, each configured for aerosolizing a different liquid.
R53. The replacement part according to the preceding embodiment and with the features of R1d, wherein the at least one reservoir are at least two reservoirs, wherein each reservoir is configured for holding a different liquid to be aerosolized.
R54. The replacement part according to any of the two preceding embodiments, wherein one of the liquids comprises at least one substance selected from nicotine and the active pharmaceutical ingredient, and one of the liquids does not comprise said substance.
R55. The replacement part according to any of the preceding replacement part embodiments with the features of R52, wherein one of the aerosolization systems comprises a heating unit and is configured for thermal aerosolization, and the other is configured for nebulization.
R56. The replacement part according to any of the preceding replacement part embodiments with the features of R26 and R52, wherein the data storage component (24) is configured for storing identification data for at least one of each of the aerosolization systems and each of the reservoirs.
R57. The replacement part according to any of the preceding replacement part embodiments and with the features of R27, wherein the data storage component (24) is configured for storing state data for at least one of each of the aerosolization systems and each of the reservoirs.
R58. The replacement part according to any of the preceding replacement part embodiments and with the features of R52 and R2, wherein each of the aerosolization systems is configured for being independently activated by the base of the mobile inhaler, wherein the base of the mobile inhaler is preferably according to M77.
R59. The replacement part according to the preceding embodiment, wherein the replacement part is configured for outputting an indicator of the different aerosolization systems being activated.
R60. The replacement part according to any of the preceding replacement part embodiments and with the features of R38 and R57, wherein the replacement part, preferably the data storage component, is configured for receiving data relating to an amount of dispensed substance from each of the two reservoirs of the replacement part.
R61. The replacement part according to any of the preceding replacement part embodiments and with the features of R57 and R39, wherein the replacement part, preferably the data storage component, is configured for receiving data relating to the use of each of the aerosolization systems of the replacement part.
R62. The replacement part according to any of the preceding replacement part embodiments with the features of R57, wherein the data storage component (24) is configured for receiving **state data** for at least one of **each** of the aerosolization systems and each of the reservoirs.
R63. The replacement part according to any of the preceding replacement part embodiments with the features of at least one of R2 and R7, wherein the replacement part is configured for receiving **state data** for at least one of **each** of the aerosolization systems and each of the reservoirs from the remainder of the mobile inhaler and/or the base of the mobile inhaler.

Below, computer program product embodiments will be discussed. These embodiments are abbreviated by the letter "P" followed by a number. Whenever reference is herein made to "computer program product embodiments", these embodiments are meant.
P1. A computer program product comprising instructions causing the mobile inhaler according to any of the system embodiments to perform the steps for which the mobile inhaler and/or its components are configured.
P2. A computer program product comprising instructions causing the base of the mobile inhaler according to any of the inhaler base embodiments to perform the steps for which the base of the mobile inhaler and/or its components are configured.
P3. A computer program product comprising instructions causing the data storage component according to at least one of
   (a) any of the system embodiment with the features of S39,
   (b) any of the inhaler base embodiments with the features of M42, and
   (c) any of the replacement part embodiments with the features of R25 to perform the steps for which the data storage component is configured.

### Brief figure description

Fig. 1 shows a first embodiment of a mobile inhaler.
Fig. 2 shows a second embodiment of the mobile inhaler.
Fig. 3 shows an embodiment of a replacement part.
Fig. 4 shows an embodiment of a data storage component.
Fig. 5 shows an embodiment of the replacement part.

### Detailed figure description

In the following, the figures will be described in detail. It is to be noted that in the figures, like features may be indicated by same reference numerals. Some features may be omitted in some figures for the sake of clarity. Also, some of the shown features may not be present in each embodiment of the invention.

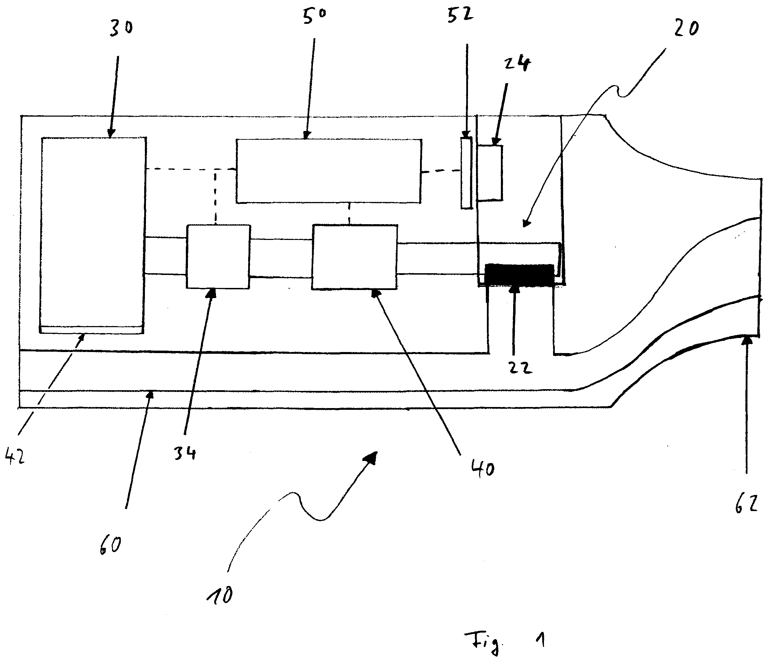

Figure 1 shows a mobile inhaler 10. The mobile inhaler 10 can be configured for aerosolization of at least one liquid, particularly of a liquid containing nicotine. The mobile inhaler 10 can be more particularly configured for vaporizing the at least one liquid.

The mobile inhaler 10 comprises a power source 30. The power source 30 can comprise a battery, such as a lithium-ion battery. Lithium-ion batteries have been a common power source for mobile, particularly handheld, devices in the past years, due to their high specific energy as well as their high energy density.

The mobile inhaler may further comprise an aerosolization system 20. The aerosolization system 20 may be configured for aerosolizing a liquid. The mobile inhaler 10 may comprise at least one reservoir configured for holding this liquid. The aerosolization system 20 may be configured heating the liquid, and more particularly for vaporizing the liquid. The aerosolization system 20 can be a vaporization system. The aerosolization system can comprise a heating element 22. The heating element 22 can be configured for converting electric energy in heat. For example, the heating element 22 can comprise a coil and/or a ceramic heating element.

However, the aerosolization system 20 may also comprise a component configured for non-thermal aerosolization of the liquid, e.g. a nebulizer.

The mobile inhaler 10 may further comprise an aerosolization system control component 40. The aerosolization system control component 40 may be configured for controlling the corresponding aerosolization system 20. The aerosolization system control component 40 may for example comprise en electronic control component for controlling a voltage, current or power provided to the heating element 22 of the aerosolization system 20. The aerosolization system control component 40 can comprise a portion of a data-processing system, such as a portion of a micro-controller. The aerosolization system control component 40 can generate control signals, e.g. for the electronic control component, or for the aerosolization system 20. The electronic control component can comprise a transistor configured for transferring and/or interrupting electric power provided by the power source 30 to the aerosolization system 20.

The mobile inhaler may further comprise a voltage control component 34. The voltage control component 34 can be configured for sensing a voltage provided by the power source 30. The voltage control component 34 can be configured for controlling the voltage provided by the power source 30. The voltage control component 34 can be configured for generating control signals for other components of the mobile inhaler 10, e.g. the aerosolization system control component 40, in order to reduce, maintain or increase the voltage provided by the power source 30 to the aerosolization system 20.

The mobile inhaler may comprise a replacement part. The replacement part may comprise the aerosolization system 20. The replacement part may also comprise the reservoir. The replacement part in Fig. 1 comprises the aerosolization system 20 with the heating element 22 and a reservoir for holding the liquid to be aerosolized.

The replacement part may be configured for being replaced by a user. For example, the replacement part may be configured for being replaced by a user without tools. The replacement part may comprise parts that need to be regularly changed, such as at least one of the aerosolization system 20, the heating element 22 and the reservoir. Even thought the reservoir does not need to be regularly changed, its contents need to be regularly changed, in other words: the liquid to be aerosolized needs to be refilled.

The replacement part may comprise an data storage component 24. The data storage component 24 may be configured for identifying the replacement part. For example, it may comprise a serial number of the replacement part. The data storage component 24 may also comprise encrypted or cryptographically signed information.

The mobile inhaler may comprise a data-transmission component 52. The data-transmission component may be configured for data transmission to and/or from the data storage component 24. The data transmission may be performed by means of electro-magnetic radiation, such as by near field communication. In such a case, the data storage component 24 may be configured for communication by electro-magnetic radiation, that is, for wireless communication. The data storage component 24 may in such cases for example comprise an RFID-tag.

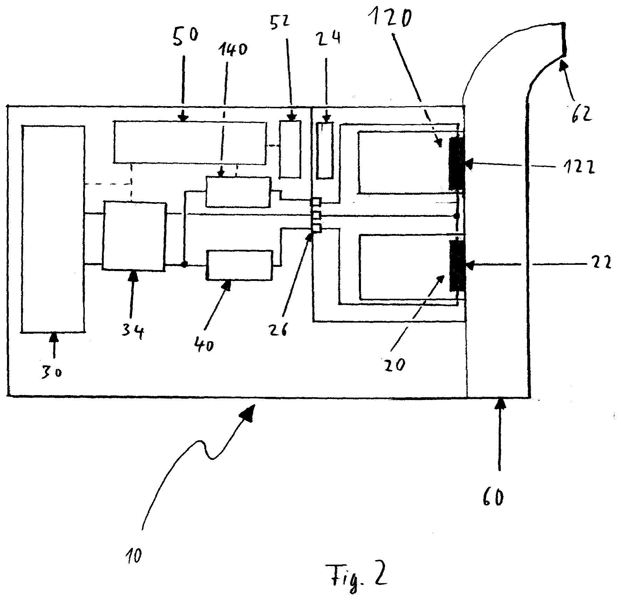

The data transmission between the data-transmission component 52 and the data storage component 24 may also be performed by means of an electric connection, as will be discussed in the context of figure 2.

The mobile inhaler 10 may further comprise a control component 50. The control component 50 may comprise a micro-controller. The control component 50 can also comprise a micro-processor. The control component 50 can be configured for performing a function of other control components of the mobile inhaler 10, such a part of the function of the data-transmission component 52, the aerosolization system control component 40, the voltage control component 34 and/or the fault detection component 42. In other words, one, some or all of these components can comprise a portion of the control component 50.

The mobile inhaler 10 may further comprise an air canal 60. The aerosolization system 20 may be configured to provide the liquid (optionally in gaseous or partially gaseous state) to the canal.

The mobile inhaler 10 may comprise a mouth piece 62. The air canal 60 may be configured for conducting air and the aerosolized liquid to the mouth piece 62.

The mobile inhaler 10 can be operated by a user, using it for inhalation. However, the person skilled in the art will also easily understand that the mobile inhaler can also be operated without a user interacting with it, for example during a test run in a laboratory, for technical approval tests or the like. In such cases, it may be helpful to provide for a suitable ventilation unit, which takes in air at the mouth piece 62 of the inhaler 10.

Figure 2 shows another embodiment of the mobile inhaler 10.

The mobile inhaler 10 in Fig. 2 may also comprise at least one of the power source 30, the control component 50, the data transmission component 52, the mouth piece 62, the air canal 60 and the voltage control component 34.

The mobile inhaler may also comprise the fault detection component 42 (not shown in Fig. 2).

The mobile inhaler 10 may comprise a first and a second aerosolization system 20, 120. Each aerosolization system 20, 120 may comprise a heating element 22, 122, as discussed above. In the example of Fig. 2, the inhaler 10 further comprises two reservoirs, each associated with one of the aerosolization systems 20, 120. Each of the reservoirs may comprise a different liquid, and each aerosolization system 20, 120 may be configured for aerosolizing the respective liquid.

The replacement part may comprise the aerosolization systems 20, 120. The replacement part may also comprise the reservoirs. The replacement part may also comprise the reservoirs and the aerosolization systems 20, 120.

The mobile inhaler 10 in Fig. 2 can be configured for delivering a mixture of aerosols from the two liquids. The mobile inhaler 10 may be configured for delivering the aerosols from the two liquids in a determined ratio. The mobile inhaler 10 may also be configured to deliver a determined amount of at least one of the two liquids, or a component thereof.

For example, the mobile inhaler 10 may be configured as a nicotine weaning device. In such a case, the mobile inhaler 10 may deliver a mixture of two liquids, one comprising nicotine, the other preferably comprising no nicotine or at least substantially no nicotine. In this case, the mobile inhaler 10 may be configured for delivering a determined amount of nicotine, or a determined amount of the liquid comprising nicotine. A delivery of the other liquid may be determined. The delivery of the other liquid may however also not be determined, e.g. if the other liquid is delivered as long as the user draws on the mobile inhaler 10 and/or as long as the user pushes a button to activate the mobile inhaler 10.

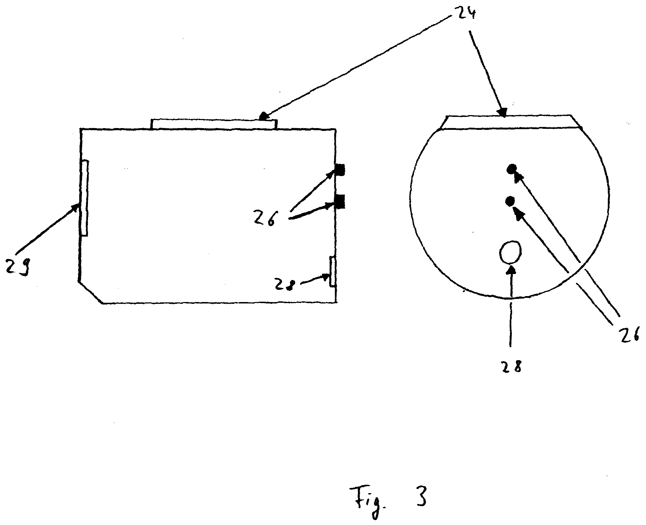

Figure 3 shows an embodiment of the replacement part in lateral view and front view. In the example of Fig. 3, the replacement part comprises an air inlet 28 and an air outlet 29.

The replacement part may comprise the aerosolization system 20 or the aerosolization systems 20, 120. The replacement part may comprise a portion of the air canal 60. This portion may extend from the air inlet 28 to the air outlet 29. However, the air inlet 28 may also be separate from the air canal 60, so that the air outlet 29 leads to the air canal.

The replacement part may also comprise the reservoir or the reservoirs.

The replacement part in Fig. 3 comprises the an aerosolization system 20 and contacts 26 configured to electrically couple the replacement part to a remainder of the mobile inhaler 10, which remainder can be a base of the mobile inhaler 10.

The replacement part in Fig. 3 further comprises the data storage component 24.

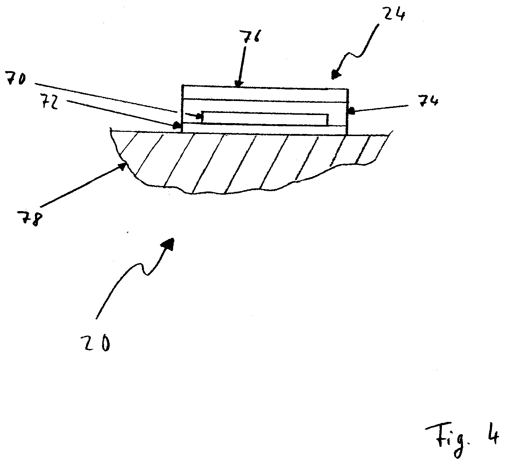

Figure 4 shows a specific embodiment of the data storage component 24, together with a replacement part. The replacement part may be the replacement part from Fig. 3.

In Fig. 4, a housing 78 of the replacement part can be seen. The data storage component 24 may be attached to the housing 78. Particularly, the data storage component 24 may comprise an adhesive layer 72 which is configured for attaching the data storage component 24 to the housing 78. Furthermore, the data storage component 24 may comprise an identification unit 70.

In Fig. 4, the identification unit 70 is an RFID-tag. The adhesive layer 72 may also be configured to attach the identification unit 70 to the housing 78.

Another adhesive layer 74 may be configured for attaching a surface layer 76 to the data storage component 24. The surface layer 76 may be configured for protecting the identification unit 70 from scratches and the like.

The surface layer 76 can for example be a film made of polymer, such as PET. For easier mounting and visual control, the film can be transparent.

The RFID tag may comprise an antenna and an integrated circuit, as the person skilled in the art easily understands.

The adhesive layers 72, 74 may comprise an acrylic adhesive. An advantage can be wide availability as well as excellent adhesive properties. The acrylic adhesive may be an adhesive transfer tape. This can be optionally advantageous for simple manufacturing of the data storage component.

At least one of the adhesive layers 72, 74 can be configured so as to destroy the identification unit 70, when it is separated from the identification unit. This can for example be achieved by using an identification unit 70 with a lower mechanical resistance than one or both of the adhesive layers 72, 74. A suitable layer 72, 74 can for example be made up of 3M (TM) Adhesive Transfer Tape 467MP.

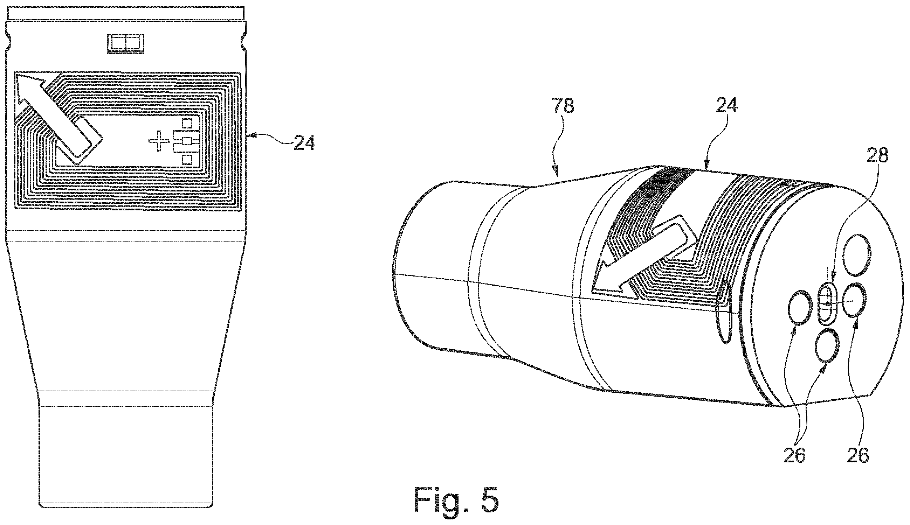

Fig. 5 shows a further embodiment of the replacement part. In Fig. 5, the data storage component 24 is mounted to the housing 78 of the replacement part. As discussed above, the data storage component 24 can comprise an RFID-tag.

The replacement part in Fig. 5 can further comprise contacts 26, as discussed above, as well as an air inlet 28. The replacement part in Fig. can also comprise at least one or two aerosolization systems 20, 120 (not shown).

### Numbered reference elements

- 10: mobile inhaler
- 20, 120: aerosolization system
- 22, 122: heating element
- 24: data storage component
- 26: contacts
- 28: air inlet
- 29: air outlet
- 30: power source
- 34: voltage control component
- 40: aerosolization system control component
- 42: fault detection component
- 50: control component
- 52: data transmission component
- 60: air canal
- 62: mouth piece
- 70: identification unit
- 72, 74: adhesive layer
- 76: surface layer
- 78: housing

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

While in the above, a preferred embodiment has been described with reference to the accompanying drawings, the skilled person will understand that this embodiment was provided for illustrative purpose only and should by no means be construed to limit the scope of the present invention, which is defined by the claims.

## Claims

1. A mobile inhaler (10), comprising a power source (30), at least one aerosolization system (20), an aerosolization system control component (40), and a data transmission component (52),
wherein each aerosolization system comprises at least one reservoir, and wherein each of the at least one reservoir is configured for holding a liquid to be aerosolized,
wherein the mobile inhaler comprises a fault detection component (42),
wherein the fault detection component (42) is configured for detecting a fault, wherein the fault detection component (42) is configured for at least one of deactivating the at least one aerosolization system and deactivating the power source (30), and
wherein the fault detection component (42) is configured for operating based on a temperature,
wherein the fault detection component is configured for performing the deactivating upon detection of the fault.

2. The mobile inhaler according to the preceding claim, wherein the mobile inhaler further comprises a voltage control component (34),
wherein the mobile inhaler is configured for operating according to an operation mode depending on a voltage provided by the power source (30), and
wherein the voltage control component (34) is configured for sensing a voltage provided by the power source (30), and, wherein the voltage control component (34) is configured for at least one of
(a) deactivating the aerosolization system, if the voltage provided by the power source (30) is below a lower threshold, and
(b) reducing a voltage provided to the at least one aerosolization system, if the voltage provided by the power source (30) is above an upper threshold.

3. The mobile inhaler according to any of the preceding claims, wherein the mobile inhaler comprises a replacement part,
wherein the replacement part comprises the at least one aerosolization system and a data storage component (24),
wherein the data transmission component (52) is configured for receiving at least one of identification data and state data from the replacement part, and for sending data to the replacement part,
wherein particularly the data storage component comprises an identification unit (70), and wherein the identification unit (70) is configured to be destroyed when removed from the replacement part.

4. The mobile inhaler according to the preceding claim, wherein the data storage component (24) is configured for storing state data, and wherein the mobile inhaler is configured for activating the replacement part only if the state data match a state-criterion.

5. The mobile inhaler according to any of the claims 3-4,
wherein the data storage component (24) is configured for storing identification data, and wherein the mobile inhaler is configured for activating the replacement part only if the identification data match an identity-criterion.

6. The mobile inhaler according to any of the preceding claims 3-5, wherein the at least one aerosolization system is at least two aerosolization systems, each configured for aerosolizing a different liquid,
wherein the at least one reservoir is at least two reservoirs, wherein each reservoir is configured for holding a different liquid to be aerosolized, and
wherein the replacement part comprises the at least two aerosolization systems and the at least two reservoirs.

7. The mobile inhaler according to the preceding claim,
wherein the data storage component (24) is configured for storing identification data for at least one of each of the aerosolization systems and each of the reservoirs, and
wherein the mobile inhaler is configured for activating each aerosolization system with the corresponding reservoir only if the identification data corresponding to the at least two aerosolization systems match the identification-criterion.

8. The mobile inhaler according to any of the claims 6-7, wherein the data storage component (24) is configured for storing state data for at least one of each of the aerosolization systems and each of the reservoirs, and
wherein the mobile inhaler is configured for activating each aerosolization system with the corresponding reservoir only if the state data corresponding to the at least two aerosolization systems match the state-criterion.

9. The mobile inhaler according to any of the claims 4-8 as far as dependent on claim 4, wherein the data transmission component (52) is configured for sending state data to the replacement part which do not match the state-criterion based on at least one of
(a) data relating to an amount of dispensed substance, and
(b) data relating to a use of the aerosolization system.

10. The mobile inhaler according to any of the claims 4-9 , wherein the data transmission component (52) is configured for sending state data to the replacement part which do not match the state-criterion if the fault detection component (42) deactivates the aerosolization system.

11. A base of a mobile inhaler (10), comprising a power source (30), a data transmission component (52), and an aerosolization system control component (40), wherein the base is configured to be connected to at least one aerosolization system (20), wherein each aerosolization system comprises at least one reservoir, and wherein each reservoir is configured for holding a liquid to be aerosolized, wherein the base comprises a fault detection component (42),
wherein the fault detection component (42) is configured for detecting a fault, and for at least one of deactivating the aerosolization system and deactivating the power source (30), and
wherein the fault detection component (42) is configured for operating based on a temperature,
wherein the fault detection component is configured for performing the deactivating upon detection of the fault.

12. The base according to claim 11, wherein the base of the mobile inhaler further comprises a voltage control component (34),
wherein the voltage control component (34) is configured for sensing a voltage provided by the power source (30),
wherein the base is configured for operating according to an operation mode depending on a voltage provided by the power source (30), and
wherein the voltage control component (34) is configured for at least one of
(a) deactivating the aerosolization system, if the voltage provided by the power source (30) is below a lower threshold, and
(b) reducing a voltage provided to the at least one aerosolization system, if the voltage provided by the power source (30) is above an upper threshold.

13. The base according to any of claims 11-12, wherein the base of the mobile inhaler is configured to be connected to a replacement part,
wherein the replacement part comprises the at least one aerosolization system and a data storage component (24),
wherein the data transmission component (52) is configured for receiving at least one of identification data and state data from the replacement part.

14. The base according to the preceding claim, wherein the base is configured for activating the replacement part only if the state data match a state-criterion.

15. The base according to any of the claims 13-14, wherein the at least one aerosolization system is at least two aerosolization systems, each configured for aerosolizing a different liquid, and wherein the at least one reservoir are at least two reservoirs, wherein each reservoir is configured for holding a different liquid to be aerosolized, and wherein the replacement part comprises the at least two aerosolization systems.

## Patentansprüche

1. Mobiler Inhalator (10), umfassend eine Stromquelle (30), mindestens ein Aerosolisierungssystem (20), eine Aerosolisierungssystem-steuerungskomponente (40) und eine Datenübertragungskomponente (52),
wobei jedes Aerosolisierungssystem mindestens einen Behälter umfasst, und wobei jeder der mindestens einen Behälter zum Halten einer zu aerosolierenden Flüssigkeit konfiguriert ist,
wobei der mobile Inhalator eine Fehlererkennungskomponente (42) umfasst, wobei die Fehlererkennungskomponente (42) zum Erkennen eines Fehlers konfiguriert ist,
wobei die Fehlererkennungskomponente (42) für mindestens eines aus Deaktivieren des mindestens einen Aerosolisierungssystems und Deaktivieren der Stromquelle (30) konfiguriert ist, und
wobei die Fehlererkennungskomponente (42) für einen Betrieb basierend auf einer Temperatur konfiguriert ist,
wobei die Fehlererkennungskomponente zum Durchführen des Deaktivierens bei Erkennung des Fehlers konfiguriert ist.

2. Mobiler Inhalator nach dem vorhergehenden Anspruch, wobei der mobile Inhalator des Weiteren eine Spannungssteuerungskomponente (34) umfasst,
wobei der mobile Inhalator für einen Betrieb gemäß einem Betriebsmodus abhängig von einer von der Stromquelle (30) bereitgestellten Spannung konfiguriert ist, und
wobei die Spannungssteuerungskomponente (34) zum Erfassen einer von der Stromquelle (30) bereitgestellten Spannung konfiguriert ist, und wobei die Spannungssteuerungskomponente (34) für mindestens eines aus Folgendem konfiguriert ist:
(a) Deaktivieren des Aerosolisierungssystems, wenn die von der Stromquelle (30) bereitgestellte Spannung unter einem unteren Schwellenwert liegt, und
(b) Reduzieren einer dem mindestens einen Aerosolisierungssystem bereitgestellten Spannung, wenn die von der Stromquelle (30) bereitgestellte Spannung über einem oberen Schwellenwert liegt.

3. Mobiler Inhalator nach einem der vorhergehenden Ansprüche, wobei der mobile Inhalator ein Austauschteil umfasst,
wobei das Austauschteil das mindestens eine Aerosolisierungssystem und eine Datenspeicherkomponente (24) umfasst,
wobei die Datenübertragungskomponente (52) zum Empfangen von mindestens einem von Identifikationsdaten und Zustandsdaten von dem Austauschteil und zum Senden von Daten an das Austauschteil konfiguriert ist,
wobei insbesondere die Datenspeicherkomponente eine Identifikationseinheit (70) umfasst, und wobei die Identifikationseinheit (70) dazu konfiguriert ist, zerstört zu werden, wenn sie von dem Austauschteil entfernt wird.

4. Mobiler Inhalator nach dem vorhergehenden Anspruch, wobei die Datenspeicherkomponente (24) zum Speichern von Zustandsdaten konfiguriert ist, und wobei der mobile Inhalator zum Aktivieren des Austauschteils nur dann konfiguriert ist, wenn die Zustandsdaten mit einem Zustandskriterium übereinstimmen.

5. Mobiler Inhalator nach einem der Ansprüche 3 bis 4,
wobei die Datenspeicherkomponente (24) zum Speichern von Identifikationsdaten konfiguriert ist, und wobei der mobile Inhalator zum Aktivieren des Austauschteils nur dann konfiguriert ist, wenn die Identifikationsdaten mit einem Identitätskriterium übereinstimmen.

6. Mobiler Inhalator nach einem der Ansprüche 3 bis 5, wobei es sich bei dem mindestens einen Aerosolisierungssystem um mindestens zwei Aerosolisierungssysteme handelt, die jeweils zum Aerosolieren einer anderen Flüssigkeit konfiguriert sind,
wobei es sich bei dem mindestens einen Behälter um mindestens zwei Behälter handelt, wobei jeder Behälter zum Halten einer anderen zu aerosolierenden Flüssigkeit konfiguriert ist, und
wobei das Austauschteil die mindestens zwei Aerosolisierungssysteme und die mindestens zwei Behälter umfasst.

7. Mobiler Inhalator nach dem vorhergehenden Anspruch,
wobei die Datenspeicherkomponente (24) zum Speichern von Identifikationsdaten für mindestens eines von jedem der Aerosolisierungssysteme und jedem der Behälter konfiguriert ist, und
wobei der mobile Inhalator zum Aktivieren jedes Aerosolisierungssystems mit dem entsprechenden Behälter nur dann konfiguriert ist, wenn die den mindestens zwei Aerosolisierungssystemen entsprechenden Identifikationsdaten mit dem Identifikationskriterium übereinstimmen.

8. Mobiler Inhalator nach einem der Ansprüche 6 bis 7, wobei die Datenspeicherkomponente (24) zum Speichern von Zustandsdaten für mindestens eines von jedem der Aerosolisierungssysteme und jeden der Behälter konfiguriert ist, und
wobei der mobile Inhalator zum Aktivieren jedes Aerosolisierungssystems mit dem entsprechenden Behälter nur dann konfiguriert ist, wenn die den mindestens zwei Aerosolisierungssystemen entsprechenden Zustandsdaten mit dem Zustandskriterium übereinstimmen.

9. Mobiler Inhalator nach einem der Ansprüche 4 bis 8, soweit abhängig von Anspruch 4, wobei die Datenübertragungskomponente (52) zum Senden von Zustandsdaten an das Austauschteil konfiguriert ist, die nicht mit dem Zustandskriterium übereinstimmen, basierend auf mindestens einem von
(a) Daten bezüglich einer Menge der abgegebenen Substanz, und
(b) Daten bezüglich einer Verwendung des Aerosolisierungssystems.

10. Mobiler Inhalator nach einem der Ansprüche 4 bis 9, wobei die Datenübertragungskomponente (52) zum Senden von Zustandsdaten an das Austauschteil konfiguriert ist, die nicht mit dem Zustandskriterium übereinstimmen, wenn die Fehlererkennungskomponente (42) das Aerosolisierungssystem deaktiviert.

11. Basis eines mobilen Inhalators (10), umfassend eine Stromquelle (30), eine Datenübertragungskomponente (52) und eine Aerosolisierungssystem-Steuerungskomponente (40), wobei die Basis dazu konfiguriert ist, mit mindestens einem Aerosolisierungssystem (20) verbunden zu werden, wobei jedes Aerosolisierungssystem mindestens einen Behälter umfasst und wobei jeder Behälter zum Halten einer zu aerosolierenden Flüssigkeit konfiguriert ist, wobei die Basis eine Fehlererkennungskomponente (42) umfasst,
wobei die Fehlererkennungskomponente (42) zum Erkennen eines Fehlers und für mindestens eines aus Deaktivieren des Aerosolisierungssystems und Deaktivieren der Stromquelle (30) konfiguriert ist, und
wobei die Fehlererkennungskomponente (42) für einen Betrieb basierend auf einer Temperatur konfiguriert ist,
wobei die Fehlererkennungskomponente zum Durchführen des Deaktivierens bei Erkennung des Fehlers konfiguriert ist.

12. Basis nach Anspruch 11, wobei die Basis des mobilen Inhalators des Weiteren eine Spannungssteuerungskomponente (34) umfasst,
wobei die Spannungssteuerungskomponente (34) zum Erfassen einer von der Stromquelle (30) bereitgestellten Spannung konfiguriert ist,
wobei die Basis für einen Betrieb gemäß einem Betriebsmodus abhängig von einer von der Stromquelle (30) bereitgestellten Spannung konfiguriert ist, und
wobei die Spannungssteuerungskomponente (34) für mindestens eines aus Folgendem konfiguriert ist:
(a) Deaktivieren des Aerosolisierungssystems, wenn die von der Stromquelle (30) bereitgestellte Spannung unter einem unteren Schwellenwert liegt, und
(b) Reduzieren einer dem mindestens einen Aerosolisierungssystem bereitgestellten Spannung, wenn die von der Stromquelle (30) bereitgestellte Spannung über einem oberen Schwellenwert liegt.

13. Basis nach einem der Ansprüche 11 bis 12, wobei die Basis des mobilen Inhalators dazu konfiguriert ist, mit einem Austauschteil verbunden zu werden,
wobei das Austauschteil das mindestens eine Aerosolisierungssystem und eine Datenspeicherkomponente (24) umfasst,
wobei die Datenübertragungskomponente (52) zum Empfangen von mindestens einem von Identifikationsdaten und Zustandsdaten von dem Austauschteil konfiguriert ist.

14. Basis nach dem vorhergehenden Anspruch, wobei die Basis zum Aktivieren des Austauschteils nur dann konfiguriert ist, wenn die Zustandsdaten mit einem Zustandskriterium übereinstimmen.

15. Basis nach einem der Ansprüche 13 bis 14, wobei es sich bei dem mindestens einen Aerosolisierungssystem um mindestens zwei Aerosolisierungssysteme handelt, die jeweils zum Aerosolieren einer anderen Flüssigkeit konfiguriert sind, und wobei es sich bei dem mindestens einen Behälter um mindestens zwei Behälter handelt, wobei jeder Behälter zum Halten einer anderen zu aerosolierenden Flüssigkeit konfiguriert ist, und wobei das Austauschteil die mindestens zwei Aerosolisierungssysteme umfasst.

## Revendications

1. Inhalateur mobile (10), comprenant une source d'alimentation (30), au moins un système d'aérosolisation (20), un composant de commande de système d'aérosolisation (40) et un composant de transmission de données (52),
dans lequel chaque système d'aérosolisation comprend au moins un réservoir, et dans lequel chacun de l'au moins un réservoir est configuré pour contenir un liquide à aérosoliser,
dans lequel l'inhalateur mobile comprend un composant de détection de défaut (42),
dans lequel le composant de détection de défaut (42) est configuré pour détecter un défaut,
dans lequel le composant de détection de défaut (42) est configuré pour au moins l'une parmi la désactivation de l'au moins un système d'aérosolisation et la désactivation de la source d'alimentation (30), et
dans lequel le composant de détection de défaut (42) est configuré pour fonctionner sur la base d'une température,
dans lequel le composant de détection de défaut est configuré pour effectuer la désactivation lors de la détection du défaut.

2. Inhalateur mobile selon la revendication précédente, dans lequel l'inhalateur mobile comprend en outre un composant de commande de tension (34),
dans lequel l'inhalateur mobile est configuré pour fonctionner selon un mode de fonctionnement dépendant d'une tension fournie par la source d'alimentation (30), et
dans lequel le composant de commande de tension (34) est configuré pour détecter une tension fournie par la source d'alimentation (30), et dans lequel le composant de commande de tension (34) est configuré pour au moins l'une parmi :
(a) la désactivation du système d'aérosolisation, si la tension fournie par la source d'alimentation (30) est au-dessous d'un seuil inférieur, et
(b) la réduction d'une tension fournie à l'au moins un système d'aérosolisation, si la tension fournie par la source d'alimentation (30) est au-dessus d'un seuil supérieur.

3. Inhalateur mobile selon l'une quelconque des revendications précédentes, dans lequel l'inhalateur mobile comprend une partie de remplacement,
dans lequel la partie de remplacement comprend l'au moins un système d'aérosolisation et un composant de stockage de données (24),
dans lequel le composant de transmission de données (52) est configuré pour recevoir au moins l'un parmi des données d'identification et des données d'état provenant de la partie de remplacement, et pour envoyer des données à la partie de remplacement,
dans lequel, en particulier, le composant de stockage de données comprend une unité d'identification (70), et dans lequel l'unité d'identification (70) est configurée pour être détruite lorsqu'elle est retirée de la partie de remplacement.

4. Inhalateur mobile selon la revendication précédente, dans lequel le composant de stockage de données (24) est configuré pour stocker des données d'état, et dans lequel l'inhalateur mobile est configuré pour activer la partie de remplacement uniquement si les données d'état correspondent à un critère d'état.

5. Inhalateur mobile selon l'une quelconque des revendications 3 à 4,
dans lequel le composant de stockage de données (24) est configuré pour stocker des données d'identification, et dans lequel l'inhalateur mobile est configuré pour activer la partie de remplacement uniquement si les données d'identification correspondent à un critère d'identité.

6. Inhalateur mobile selon l'une quelconque des revendications précédentes 3 à 5, dans lequel l'au moins un système d'aérosolisation consiste en au moins deux systèmes d'aérosolisation, chacun étant configuré pour aérosoliser un liquide différent,
dans lequel l'au moins un réservoir consiste en au moins deux réservoirs, chaque réservoir étant configuré pour contenir un liquide à aérosoliser différent, et
dans lequel la partie de remplacement comprend les au moins deux systèmes d'aérosolisation et les au moins deux réservoirs.

7. Inhalateur mobile selon la revendication précédente,
dans lequel le composant de stockage de données (24) est configuré pour stocker des données d'identification pour au moins l'un parmi chacun des systèmes d'aérosolisation et chacun des réservoirs, et
dans lequel l'inhalateur mobile est configuré pour activer chaque système d'aérosolisation avec le réservoir correspondant uniquement si les données d'identification correspondant aux au moins deux systèmes d'aérosolisation correspondent au critère d'identification.

8. Inhalateur mobile selon l'une quelconque des revendications 6 à 7, dans lequel le composant de stockage de données (24) est configuré pour stocker des données d'état pour au moins l'un parmi chacun des systèmes d'aérosolisation et chacun des réservoirs, et
dans lequel l'inhalateur mobile est configuré pour activer chaque système d'aérosolisation avec le réservoir correspondant uniquement si les données d'état correspondant aux au moins deux systèmes d'aérosolisation correspondent au critère d'état.

9. Inhalateur mobile selon l'une quelconque des revendications 4 à 8 pour autant qu'elles dépendent de la revendication 4, dans lequel le composant de transmission de données (52) est configuré pour envoyer des données d'état à la partie de remplacement qui ne correspondent pas au critère d'état sur la base d'au moins l'un parmi :
(a) des données relatives à une quantité de substance distribuée ; et
(b) des données relatives à une utilisation du système d'aérosolisation.

10. Inhalateur mobile selon l'une quelconque des revendications 4 à 9, dans lequel le composant de transmission de données (52) est configuré pour envoyer des données d'état à la partie de remplacement qui ne correspondent pas au critère d'état si le composant de détection de défaut (42) désactive le système d'aérosolisation.

11. Base d'un inhalateur mobile (10), comprenant une source d'alimentation (30), un composant de transmission de données (52) et un composant de commande de système d'aérosolisation (40), dans laquelle la base est configurée pour être reliée à au moins un système d'aérosolisation (20), chaque système d'aérosolisation comprenant au moins un réservoir, et chaque réservoir étant configuré pour contenir un liquide à aérosoliser, la base comprenant un composant de détection de défaut (42),
dans laquelle le composant de détection de défaut (42) est configuré pour détecter un défaut, et pour au moins l'une parmi la désactivation du système d'aérosolisation et la désactivation de la source d'alimentation (30), et
dans laquelle le composant de détection de défaut (42) est configuré pour fonctionner sur la base d'une température,
dans laquelle le composant de détection de défaut est configuré pour effectuer la désactivation lors de la détection du défaut.

12. Base selon la revendication 11, dans laquelle la base de l'inhalateur mobile comprend en outre un composant de commande de tension (34),
dans laquelle le composant de commande de tension (34) est configuré pour détecter une tension fournie par la source d'alimentation (30),
dans laquelle la base est configurée pour fonctionner selon un mode de fonctionnement dépendant d'une tension fournie par la source d'alimentation (30), et
dans laquelle le composant de commande de tension (34) est configuré pour au moins l'une parmi
(a) la désactivation du système d'aérosolisation, si la tension fournie par la source d'alimentation (30) est au-dessous d'un seuil inférieur, et
(b) la réduction la tension fournie à l'au moins un système d'aérosolisation, si la tension fournie par la source d'alimentation (30) est au-dessus d'un seuil supérieur.

13. Base selon l'une quelconque des revendications 11 à 12, dans laquelle la base de l'inhalateur mobile est configurée pour être reliée à une partie de remplacement,
dans laquelle la partie de remplacement comprend l'au moins un système d'aérosolisation et un composant de stockage de données (24),
dans laquelle le composant de transmission de données (52) est configuré pour recevoir au moins l'un parmi des données d'identification et des données d'état provenant de la partie de remplacement.

14. Base selon la revendication précédente, dans laquelle la base est configurée pour activer la partie de remplacement uniquement si les données d'état correspondent à un critère d'état.

15. Base selon l'une quelconque des revendications 13 à 14, dans laquelle l'au moins un système d'aérosolisation consiste en au moins deux systèmes d'aérosolisation, chacun étant configuré pour aérosoliser un liquide différent, et dans laquelle l'au moins un réservoir consiste en au moins deux réservoirs, chaque réservoir étant configuré pour contenir un liquide à aérosoliser différent, et dans laquelle la partie de remplacement comprend les au moins deux systèmes d'aérosolisation.
